# EUROPÄISCHE PATENTSCHRIFT

(11) **EP 2 768 808 B1**
(45) Veröffentlichungstag und Bekanntmachung des Hinweises auf die Patenterteilung: **15.11.2017**
(21) Anmeldenummer: 12766002.5
(22) Anmeldetag: 24.09.2012
(51) Int. Cl.: C07D 209/94, C07D 495/04

(54) **MATERIALIEN FÜR ORGANISCHE ELEKTROLUMINESZENZVORRICHTUNGEN**
MATERIALS FOR ORGANIC ELECTROLUMINESCENT DEVICES
MATÉRIAUX DESTINÉS À DES DISPOSITIFS ÉLECTROLUMINESCENTS ORGANIQUES

(30) Priorität: 20.10.2011 EP 11008426
(43) Veröffentlichungstag der Anmeldung: 27.08.2014
(73) Patentinhaber: Merck Patent GmbH, 64293 Darmstadt (DE)
(72) Erfinder: JATSCH, Anja, 60489 Frankfurt am Main (DE); ANÉMIAN, Rémi, Manouk, Seoul 657-169 (KR); SCHROEDER, Bernd, 65606 Villmar-Weyer (DE); BUESING, Arne, 65929 Frankfurt am Main (DE); STOESSEL, Philipp, 60487 Frankfurt am Main (DE); PFLUMM, Christof, 64291 Darmstadt (DE); KROEBER, Jonas, Valentin, 60311 Frankfurt am Main (DE); EBERLE, Thomas, 76829 Landau (DE)
(86) Internationale Anmeldenummer: PCT/EP2012/003981
(87) Internationale Veröffentlichungsnummer: WO 2013/056776

(56) Entgegenhaltungen:
- WO-A1-2010/136109
- WO-A1-2011/000455
- WO-A1-2012/039561
- WO-A1-2012/095143
- WO-A2-2010/107244

## Beschreibung

Die vorliegende Erfindung beschreibt Triphenylen-Derivate, insbesondere zur Verwendung als Triplettmatrixmaterialien in organischen Elektrolumineszenzvorrichtungen. Die Erfindung betrifft ferner ein Verfahren zur Herstellung der erfindungsgemäßen Verbindungen sowie elektronische Vorrichtungen, enthaltend diese.

Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Als emittierende Materialien werden hierbei häufig metallorganische Komplexe eingesetzt, die Phosphoreszenz statt Fluoreszenz zeigen (M. A. Baldo et al., Appl. Phys. Lett. 1999, 75, 4-6). Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, insbesondere auch bei OLEDs, die Triplettemission (Phosphoreszenz) zeigen, immer noch Verbesserungsbedarf, beispielsweise im Hinblick auf Effizienz, Betriebsspannung und Lebensdauer.

Die Eigenschaften von phosphoreszierenden OLEDs werden nicht nur von den eingesetzten Triplettemittern bestimmt. Hier sind insbesondere auch die anderen verwendeten Materialien wie zum Beispiel Matrixmaterialien oder Lochblockiermaterialien von besonderer Bedeutung. Verbesserungen dieser Materialien können somit auch zu deutlichen Verbesserungen der OLED-Eigenschaften führen.

Gemäß dem Stand der Technik werden unter anderem Indolocarbazolderivate (z. B. gemäß WO 2007/063754 oder WO 2008/056746) oder Indenocarbazolderivate (z. B. gemäß WO 2010/136109 oder WO 2011/000455), insbesondere solche, die mit elektronenarmen Heteroaromaten wie Triazin substituiert sind, als Matrixmaterialien für phosphoreszierende Emitter verwendet. Weiterhin werden beispielsweise Triphenylenderivate (z. B. gemäß JP 2006/143845 oder WO 2006/047119) als Matrixmaterialien für phosphoreszierende Emitter verwendet. Allerdings besteht bei Verwendung dieser Matrixmaterialien noch Verbesserungsbedarf, insbesondere in Bezug auf die Effizienz, die Lebensdauer und die Betriebsspannung der Vorrichtung.

Aufgabe der vorliegenden Erfindung ist die Bereitstellung von Verbindungen, welche sich für den Einsatz in einer fluoreszierenden oder insbesondere in einer phosphoreszierenden OLED eignen, beispielsweise als Matrixmaterial oder als Elektronentransport- bzw. Lochblockiermaterial. Insbesondere ist es die Aufgabe der vorliegenden Erfindung, Matrixmaterialien bereitzustellen, welche sich auch für rot und grün und gegebenenfalls auch für blau phosphoreszierende OLEDs eignen, bereitzustellen und die zu guter Effizienz, hoher Lebensdauer und geringer Betriebsspannung führen. Gerade die Eigenschaften der Matrixmaterialien haben einen wesentlichen Einfluss die Lebensdauer und die Effizienz der organischen Elektrolumineszenzvorrichtung.

Es wurde überraschend gefunden, dass Elektrolumineszenzvorrichtungen, die Verbindungen gemäß der folgenden Formel (1), Formel (2), Formel (3) oder Formel (4) enthalten, Verbesserungen gegenüber dem Stand der Technik aufweisen, insbesondere beim Einsatz als Matrixmaterialien für phosphoreszierende Dotanden, aber auch bei Verwendung als Elektronentransport- oder als Lochblockierverbindungen.

Gegenstand der vorliegenden Erfindung ist daher eine Verbindung der folgenden Formel (1), 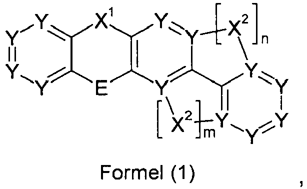 wobei für die verwendeten Symbole und Indizes gilt:
- Y: ist C, wenn an diese Gruppe Y eine Gruppe X² gebunden ist, bzw. ist bei jedem Auftreten gleich oder verschieden CR, wenn an diese Gruppe Y keine Gruppe X² gebunden ist;
- E: ist eine Einfachbindung;
- X¹: ist eine bivalente Brücke und steht für N(R¹);
- X²: ist eine bivalente Brücke und steht für C(R¹)₂;
oder eine Verbindung der Formel (2), Formel (3) oder Formel (4), 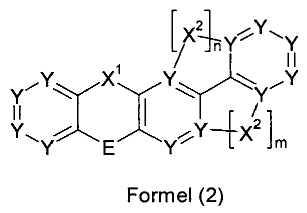 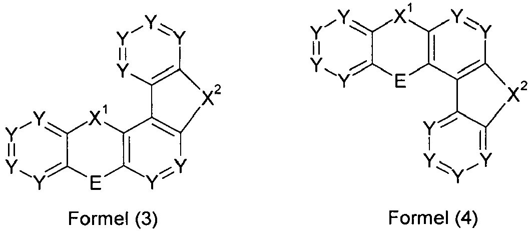 wobei für die verwendeten Symbole und Indizes gilt:
- Y: ist C, wenn an diese Gruppe Y eine Gruppe X² gebunden ist, bzw. ist bei jedem Auftreten gleich oder verschieden CR oder N, wenn an diese Gruppe Y keine Gruppe X² gebunden ist;
- E: ist eine Einfachbindung oder eine bivalente Brücke, ausgewählt aus N(R¹), C(R¹)₂, O oder S;

- X¹, X²: ist bei jedem Auftreten gleich oder verschieden eine bivalente Brücke, ausgewählt aus N(R¹), O, C(R¹)₂ oder S; mit der Maßgabe, dass X¹ und X² nicht gleichzeitig für O stehen;
weiterhin gilt für die Symbole und Indizes in den Formeln (1) bis (4):
- R: ist bei jedem Auftreten gleich oder verschieden H oder D oder die Reste R, die ungleich H oder D sind, sind ausgewählt aus der Gruppe bestehend aus N(Ar)₂, einer geradkettigen Alkylgruppe mit 1 bis 10 C-Atomen, einer verzweigten Alkylgruppe mit bis zu 10 C-Atomen oder einem aromatischen oder heteroaromatischen Ringsystem mit 5 bis 40 aromatischen Ringatomen, das jeweils durch einen oder mehrere Reste R² substituiert sein kann;
- R¹: steht, wenn E, X¹ und/oder X² für NR¹ stehen, für ein aromatisches oder heteroaromatisches Ringsystem mit 6 bis 24 aromatischen Ringatomen, das durch einen oder mehrere Reste R² substituiert sein kann; und steht, wenn E, X¹ und/oder X² für C(R¹)₂ stehen, für eine geradkettige Alkylgruppe mit 1 bis 20 C-Atomen oder für eine verzweigte oder cyclische Alkylgruppe mit 3 bis 20 C-Atomen, die jeweils mit einem oder mehreren Resten R² substituiert sein kann, wobei ein oder mehrere H-Atome durch D, F oder CN ersetzt sein können, oder für ein aromatisches oder heteroaromatisches Ringsystem mit 5 bis 60 aromatischen Ringatomen, das jeweils durch einen oder mehrere Reste R² substituiert sein kann;
- Ar: ist bei jedem Auftreten gleich oder verschieden ein aromatisches oder heteroaromatisches Ringsystem, bevorzugt eine Aryl- oder Heteroarylgruppe mit 5 bis 40 Ringatomen, das mit einem oder mehreren Resten R³ substituiert sein kann;
- R²: ist bei jedem Auftreten gleich oder verschieden H, D, F, N(Ar)₂, CN, eine geradkettige Alkylgruppe mit 1 bis 40 C-Atomen oder eine verzweigte oder cyclische Alkylgruppe mit 3 bis 40 C-Atomen, wobei ein oder mehrere H-Atome durch D, F oder CN ersetzt sein können, oder ein aromatisches oder heteroaromatisches Ringsystem mit 5 bis 60 aromatischen Ringatomen, das jeweils durch einen oder mehrere Reste R³ substituiert sein kann;
- R³: ist bei jedem Auftreten gleich oder verschieden H, D oder ein aliphatischer Kohlenwasserstoffrest mit 1 bis 20 C-Atomen oder eine Aryl- oder Heteroarylgruppe mit 5 bis 40 Ringatomen oder eine Kombination dieser Gruppen;
- n, m: sind gleich oder verschieden bei jedem Auftreten 0 oder 1, mit der Maßgabe, dass n + m = 1 oder 2 ist;
dadurch gekennzeichnet, dass mindestens eine Gruppe R und/oder R¹ vorhanden ist, die für eine Gruppe der folgenden Formel (5) steht, 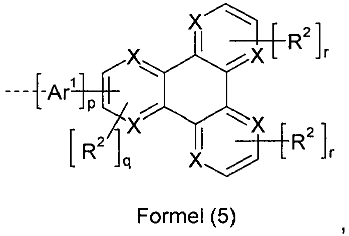 wobei die gestrichelte Bindung die Verknüpfung der Gruppe der Formel (5) andeutet, R² die oben genannten Bedeutungen aufweist und weiterhin gilt:
- X: ist C, wenn an dieses X die Gruppe Ar¹ bzw. das restliche Molekül gebunden ist, und ist bei jedem Auftreten gleich oder verschieden CR² oder N in den sonstigen Fällen;
- Ar¹: ist ein bivalentes aromatisches oder heteroaromatisches Ringsystem mit 5 bis 40 aromatischen Ringatomen, welches durch einen oder mehrere Reste R² substituiert sein kann;
- p: ist bei jedem Auftreten gleich oder verschieden 0 oder 1;
- q, r: ist bei jedem Auftreten gleich oder verschieden 0, 1 oder 2.

Eine Arylgruppe im Sinne dieser Erfindung enthält 6 bis 60 C-Atome; eine Heteroarylgruppe im Sinne dieser Erfindung enthält 2 bis 60 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte (anellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Ein aromatisches Ringsystem im Sinne dieser Erfindung enthält 6 bis 80 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C-, N- oder O-Atom, verbunden sein können. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind. Weiterhin werden miteinander durch Einfachbindung verknüpfte Aromaten, wie zum Beispiel Biphenyl, als aromatisches Ringsystem im Sinne dieser Anmeldung bezeichnet.

Im Rahmen der vorliegenden Erfindung werden unter einem aliphatischen Kohlenwasserstoffrest bzw. einer Alkylgruppe bzw. einer Alkenyl- oder Alkinylgruppe, die typischerweise 1 bis 40 oder auch 1 bis 20 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkenyl-, Alkinyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen oder heteroaromatischen Ringsystem mit 5 - 80 aromatischen Ringatomen, welches noch jeweils mit den oben genannten Resten substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombination dieser Systeme. Dabei können diese Gruppen jeweils durch die oben genannten Reste substituiert sein.

Bevorzugt sind die Verbindungen gemäß Formel (1) oder Formel (2), ganz besonders bevorzugt sind die Verbindungen gemäß Formel (1).

Bevorzugt sind weiterhin Verbindungen, in denen n + m = 1 gilt.

In einer bevorzugten Ausführungsform der erfindungsgemäßen Verbindungen ist E ausgewählt aus einer Einfachbindung oder einer bivalenten Brücke, ausgewählt aus N(R¹), C(R¹)₂ und O. Besonders bevorzugt ist E ausgewählt aus einer Einfachbindung, N(R¹) oder C(R¹)₂. Ganz besonders bevorzugt steht E für eine Einfachbindung.

In einer weiteren bevorzugten Ausführungsform der erfindungsgemäßen Verbindungen ist X¹ ausgewählt aus der Gruppe bestehend aus NR¹ oder S. Besonders bevorzugt steht X¹ für NR¹.

In einer weiteren bevorzugten Ausführungsform der erfindungsgemäßen Verbindungen ist X² ausgewählt aus der Gruppe bestehend aus C(R¹)₂, NR¹ oder S, besonders bevorzugt C(R¹)₂ oder NR¹, ganz besonders bevorzugt C(R¹)₂.

In einer besonders bevorzugten Ausführungsform der erfindungsgemäßen Verbindungen steht X¹ für N(R¹), und X² steht für C(R¹)₂. Besonders bevorzugt steht gleichzeitig E für eine Einfachbindung.

In einer weiteren bevorzugten Ausführungsform der Erfindung steht Y für C, wenn an diese Gruppe Y eine Gruppe X² gebunden ist, oder steht gleich oder verschieden bei jedem Auftreten für CR, wenn an diese Gruppe Y keine Gruppe X² gebunden ist.

In einer weiteren bevorzugten Ausführungsform steht X für CR², bzw. X steht für C, wenn an dieses X die Gruppe Ar¹ bzw. das restliche Molekül gebunden ist.

Bevorzugte Ausführungsformen der Verbindungen gemäß Formel (1), (2), (3) bzw. (4) sind die Verbindungen gemäß den folgenden Formeln (1a), (2a), (3a) bzw. (4a), 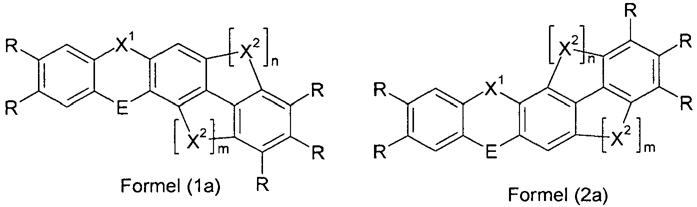 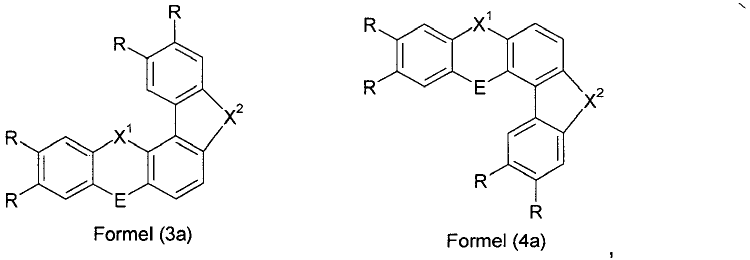 wobei die verwendeten Symbole und Indizes die oben genannten Bedeutungen aufweisen, wobei für Formel (1a) die Definitionen der Formel (1) und für Formel (2a) bis (4a) die Definitionen der Formeln (2) bis (4) gelten. Insbesondere weisen die verwendeten Symbole die oben genannten bevorzugten Bedeutungen auf.

Besonders bevorzugt sind Strukturen, in denen X¹ für NR¹ steht. Besonders bevorzugt sind daher die Strukturen der folgenden Formeln (1 b) bis (4b), 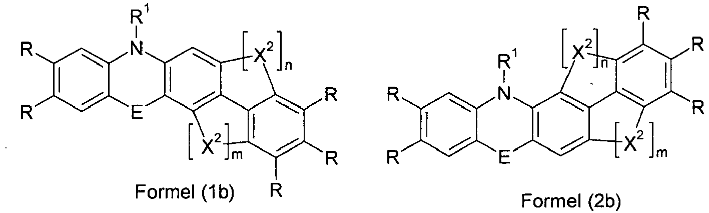 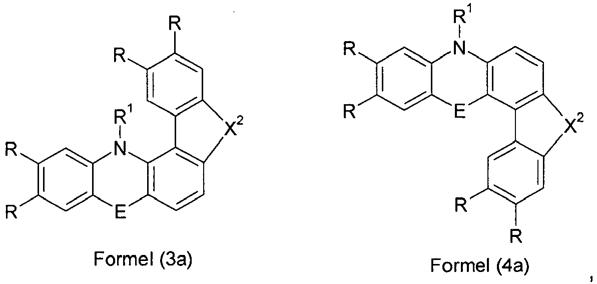 wobei die verwendeten Symbole und Indizes die oben genannten Bedeutungen aufweisen, wobei für Formel (1b) die Definitionen der Formel (1) und für Formel (2b) bis (4b) die Definitionen der Formeln (2) bis (4) gelten, insbesondere die oben genannten bevorzugten Bedeutungen.

In einer besonders bevorzugten Ausführungsform der Erfindung ist die Verbindung der Formel (1) ausgewählt aus den folgenden allgemeinen Strukturen (1e) und (1i): 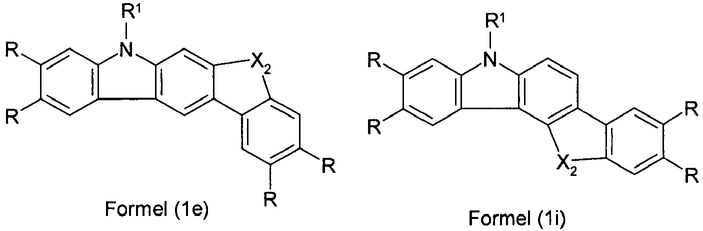 wobei die Symbole und Indizes die oben angegebenen Bedeutungen haben.

Wie oben beschrieben, enthält die erfindungsgemäße Verbindung mindestens eine Gruppe R bzw. R¹ gemäß Formel (5). In einer bevorzugten Ausführungsform der Erfindung steht mindestens eine Gruppe X¹ und/oder X² für NR¹, wobei die Gruppe R¹ für eine Gruppe der Formel (5) steht. In einer weiteren bevorzugten Ausführungsform der Erfindung steht mindestens eine Gruppe R für eine Gruppe der Formel (5).

In einer weiteren bevorzugten Ausführungsform der Erfindung enthält die Verbindung gemäß Formel (1), Formel (2), Formel (3) bzw. Formel (4) eine, zwei oder drei Gruppen gemäß Formel (5), besonders bevorzugt eine oder zwei Gruppen gemäß Formel (5), ganz besonders bevorzugt genau eine Gruppe gemäß Formel (5).

In einer weiteren bevorzugten Ausführungsform der erfindungsgemäßen Verbindungen ist die Gruppe gemäß Formel (5) eine Gruppe der folgenden Formel (5a), 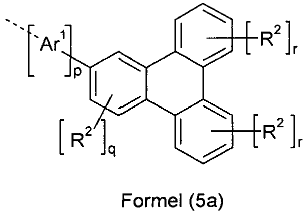 wobei die verwendeten Symbole und Indizes die oben genannten Bedeutungen aufweisen, d. h. die Triphenyleneinheit ist über die 2-Position gebunden.

In einer bevorzugten Ausführungsform der Formel (5a) ist der Index q = 0 und der Index r ist gleich oder verschieden bei jedem Auftreten 0 oder 1, besonders bevorzugt 0.

Bevorzugte Ausführungsformen der Formel (5a) sind daher die Strukturen der folgenden Formel (5b) und besonders bevorzugte Ausführungsformen sind die Strukturen der folgenden Formel (5c), 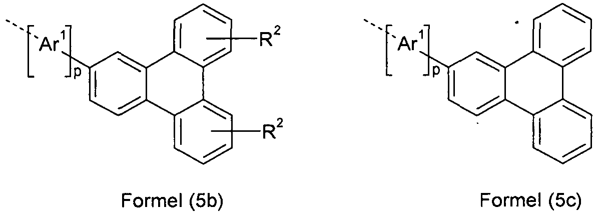 wobei die verwendeten Symbole und Indizes die oben genannten Bedeutungen aufweisen.

Dabei steht in den Gruppen der Formel (5) bzw. (5a) bis (5c) Ar¹ bevorzugt für ein bivalentes aromatisches oder heteroaromatisches Ringsystem mit 6 bis 24 aromatischen Ringatomen, welches bevorzugt keine kondensierte Aryl- oder Heteroarylgruppe mit mehr als zwei direkt aneinander kondensierten Sechsringen enthält. Bevorzugte Gruppen Ar¹ sind ausgewählt aus der Gruppe bestehend aus ortho-, meta- oder para-Phenylen, ortho-, meta- oder para-Biphenyl, ortho-, meta- oder para-Terphenyl, ortho-, meta- oder para-Quaterphenyl, Furan, Benzofuran, Dibenzofuran, Pyrrol, Indol, Carbazol oder Dibenzothiophen.

Die oben genannten Ausführungsformen der Erfindung sind beliebig miteinander kombinierbar. Insbesondere sind die oben aufgeführten allgemeinen Formeln (1), (2), (3) bzw. (4) bzw. die bevorzugten Ausführungsformen beliebig mit den Formeln (5a) bzw. (5b) bzw. (5c) sowie mit den oben genannten bevorzugten Ausführungsformen für die weiteren Symbole und Indizes kombinierbar. In einer bevorzugten Ausführungsform der Erfindung treten mehrere oder alle der oben genannten Bevorzugungen gleichzeitig auf.

In einer bevorzugten Ausführungsform der Erfindung ist Ar bzw. R, wenn R für ein aromatisches oder heteroaromatisches Ringsystem steht, ausgewählt aus substituiertem oder unsubstituiertem Phenyl, Naphthyl, Pyridin, Triazin, Pyrimidin, Benzimidazol, Thiophen, Triphenylamin oder Kombinationen dieser Gruppen, welche jeweils mit einem oder mehreren Resten R² substituiert sein können.

Beispiele für erfindungsgemäße Verbindungen der Formel (1), Formel (2), Formel (3) und Formel (4) sind die nachstehend gezeigten Strukturen.

| | |
|---|---|
| 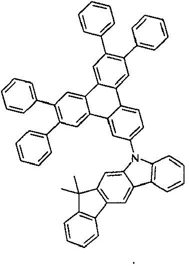 | 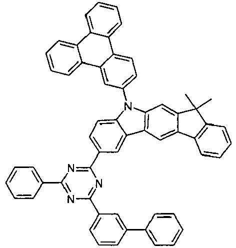 |
| (1) | (2) |
| 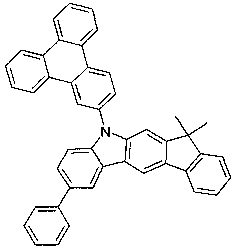 | 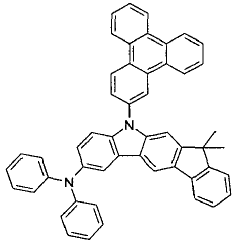 |
| (3) | (4) |
| 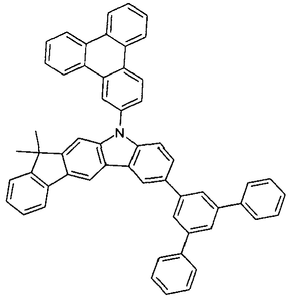 | |
| (7) | |
| 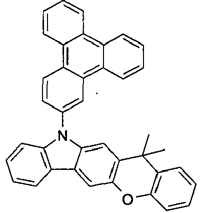 | 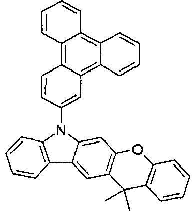 |
| (11) | (12) |
| 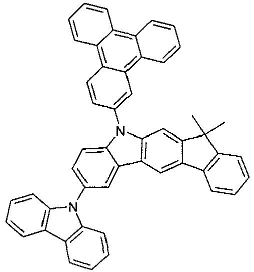 | 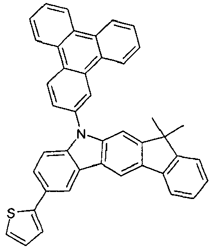 |
| (13) | (14) |
| 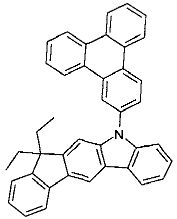 | 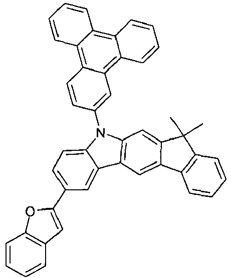 |
| (15) | (16) |
| | 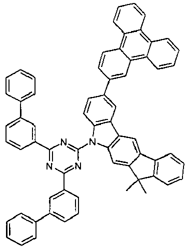 |
| | (18) |
| 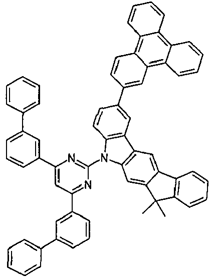 | 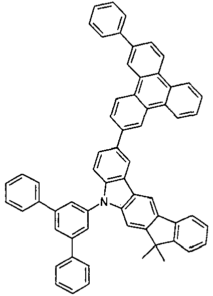 |
| (19) | (20) |
| 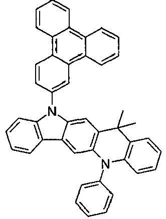 | 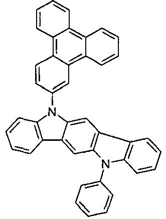 |
| (21) | (22) |
| 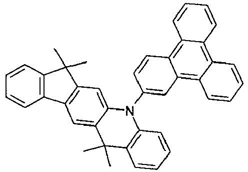 | 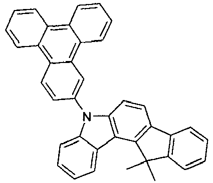 |
| (23) | (24) |
| 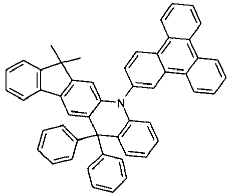 | 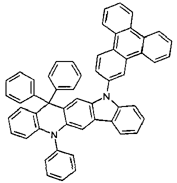 |
| (25) | (26) |
| 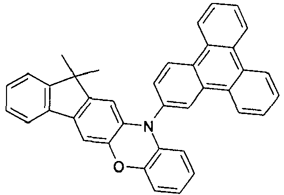 | |
| (27) | |
| 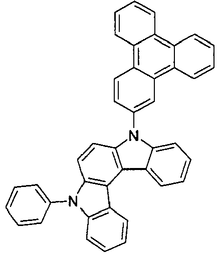 | 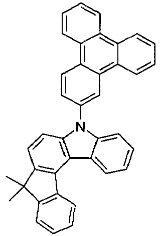 |
| (33) | (34) |
| 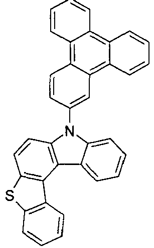 | |
| (35) | |
| 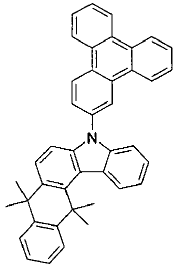 | 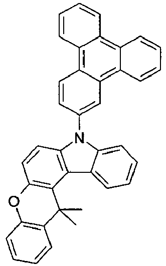 |
| (37) | (38) |
| 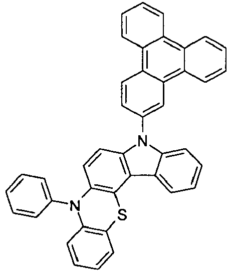 | 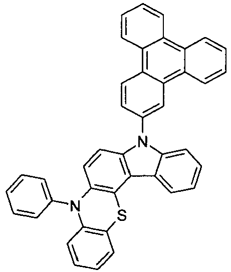 |
| (39) | (40) |
| 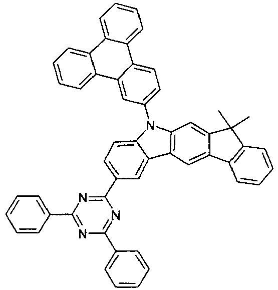 | 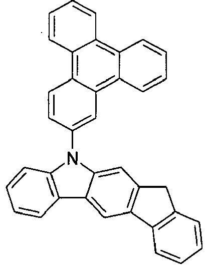 |
| (47) | (48) |
| | 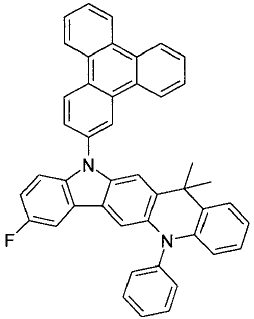 |
| | (50) |
| | 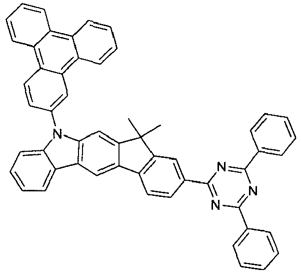 |
| | (52) |
| 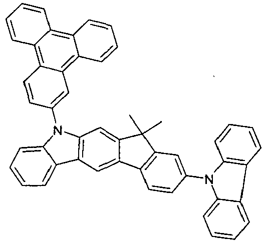 | 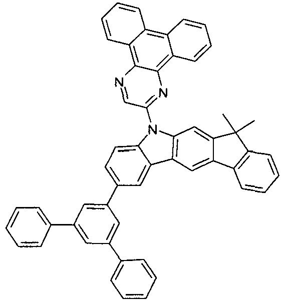 |
| (53) | (54) |
| 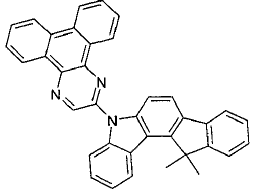 | |
| (55) | |
| 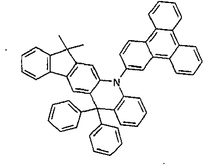 | 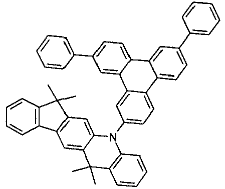 |
| (57) | (58) |
| | 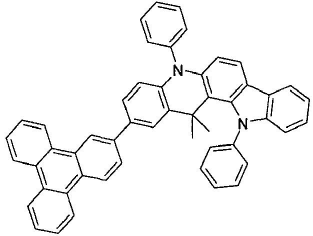 |
| | (60) |
| | 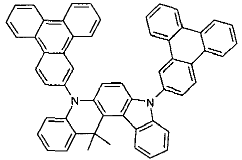 |
| | (62) |
| 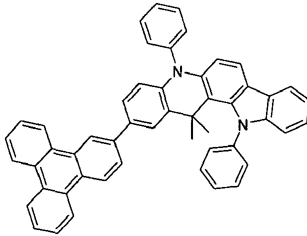 | 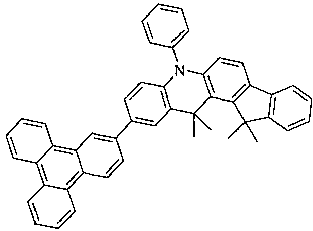 |
| (63) | (64) |
| 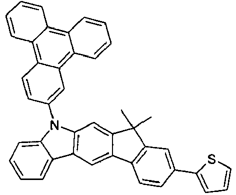 | 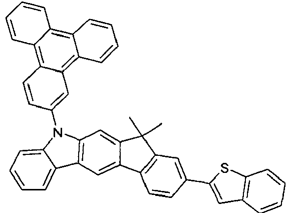 |
| (65) | (66) |
| 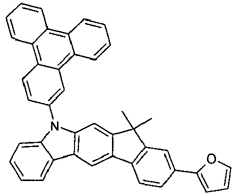 | 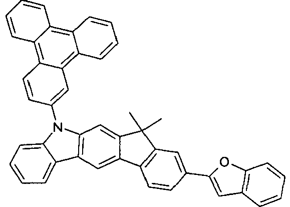 |
| (67) | (68) |
| 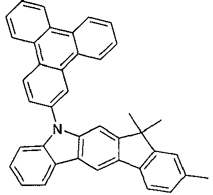 | 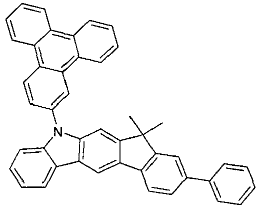 |
| (71) | (72) |
| 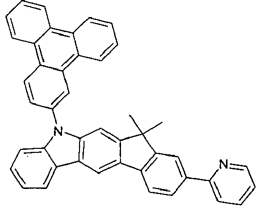 | 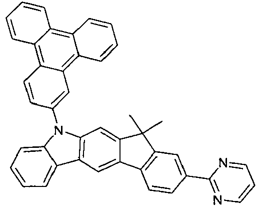 |
| (73) | (74) |
| 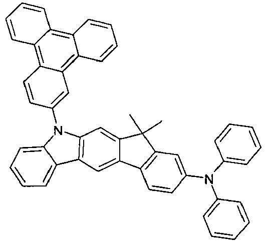 | 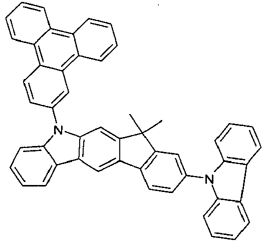 |
| (75) | (76) |
| | 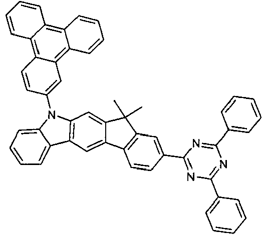 |
| | (78) |
| | 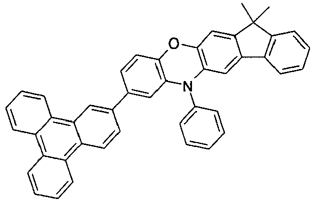 |
| | (80) |
| 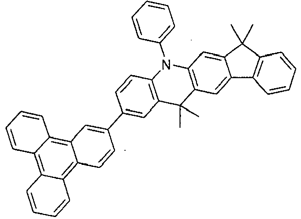 | 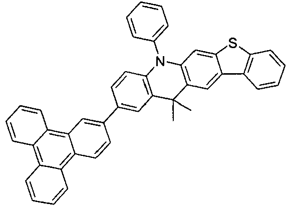 |
| (81) | (82) |
| 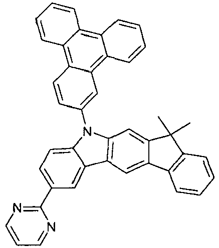 | 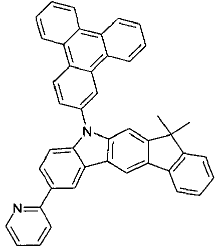 |
| (83) | (84) |
| 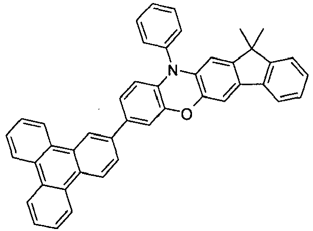 | 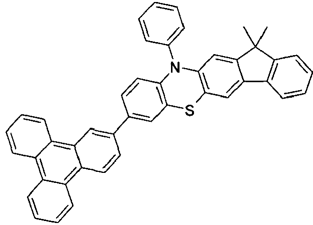 |
| (85) | (86) |
| 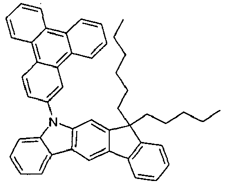 | 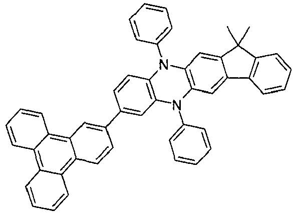 |
| (87) | (88) |
| 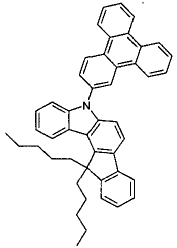 | 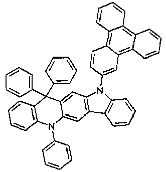 |
| (89) | (90) |
| 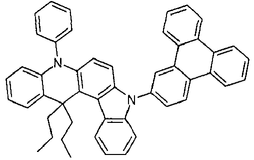 | 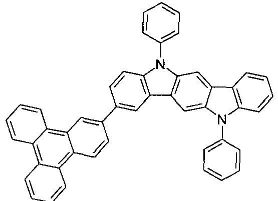 |
| (91) | (92) |
| 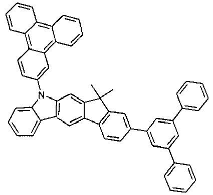 | 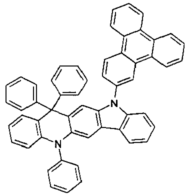 |
| (93) | (94) |
| | 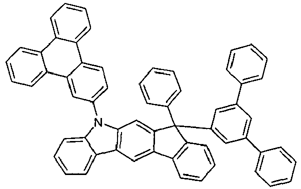 |
| | (96) |
| 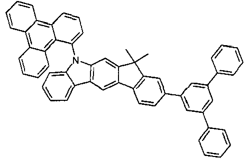 | 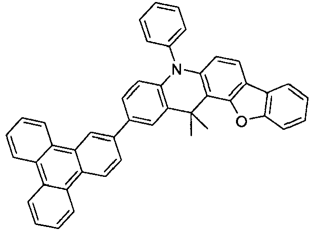 |
| (97) | (98) |
| | 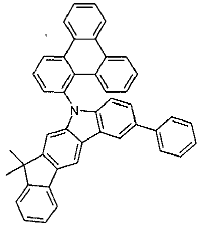 |
| | (102) |
| | 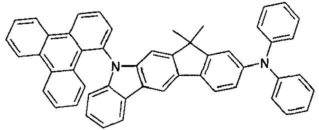 |
| | (104) |
| 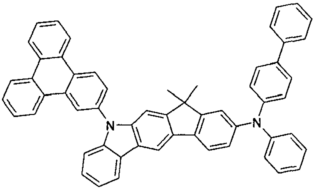 | 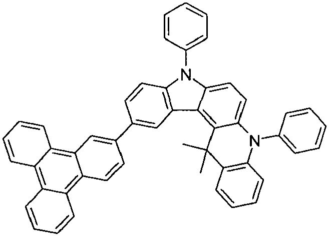 |
| (105) | (104) |
| 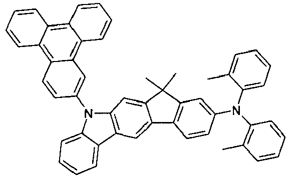 | 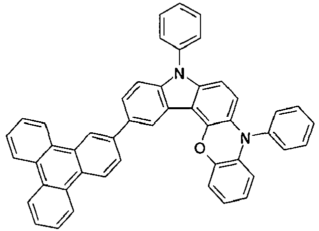 |
| (105) | (106) |
| 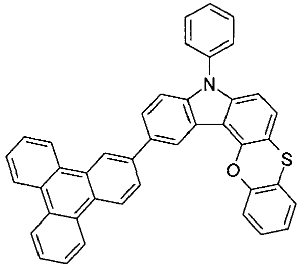 | 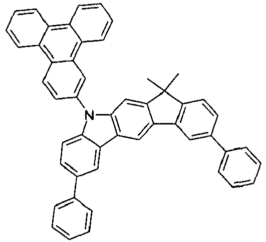 |
| (107) | (108) |
| 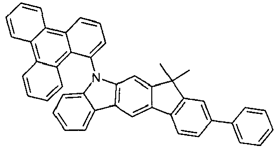 | 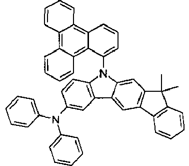 |
| (109) | (110) |
| 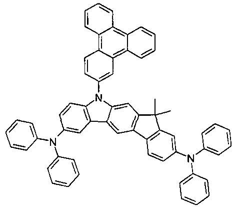 | 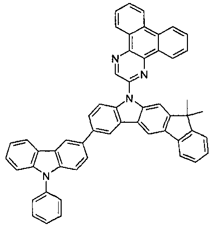 |
| (111) | (112) |
| 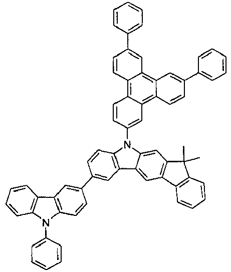 | 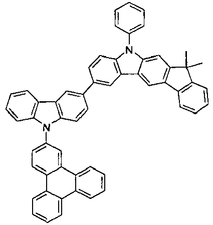 |
| (113) | (114) |
| 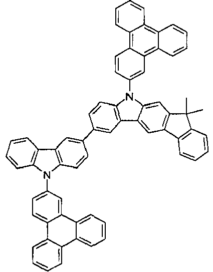 | 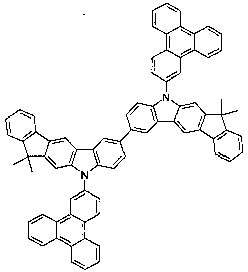 |
| (115) | (116) |
| 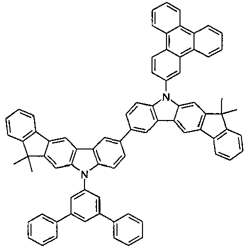 | 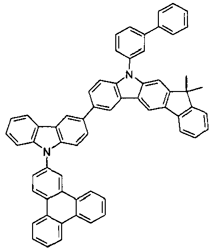 |
| (117) | (118) |
| 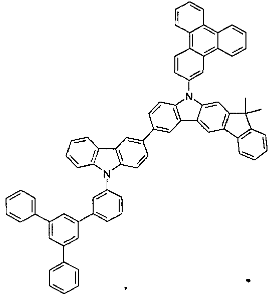 | 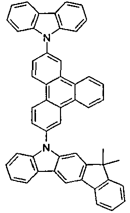 |
| (119) | (120) |
| 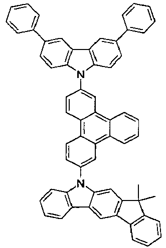 | 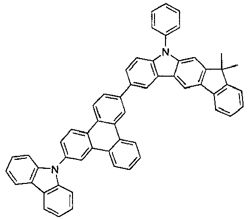 |
| (121) | (122) |
| 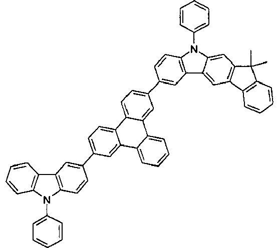 | 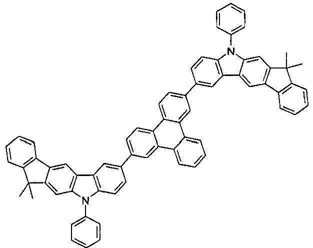 |
| (123) | (124) |
| 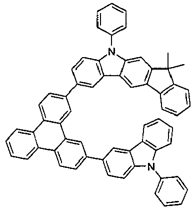 | 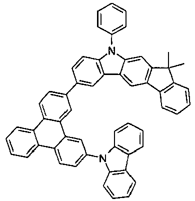 |
| (125) | (126) |
| 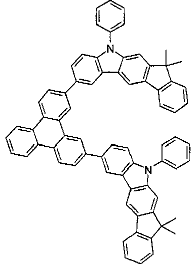 | 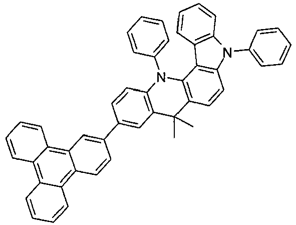 |
| (127) | (128) |
| 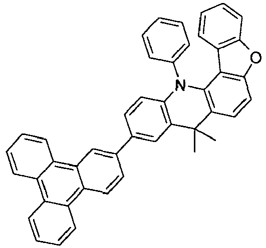 | 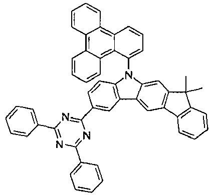 |
| (129) | (130) |
| 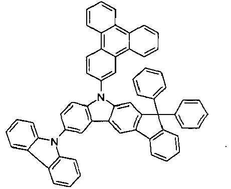 | |
| (131) | |
| 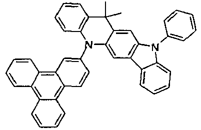 | 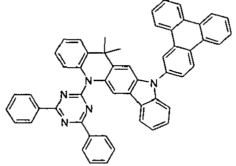 |
| (133) | (134) |
| 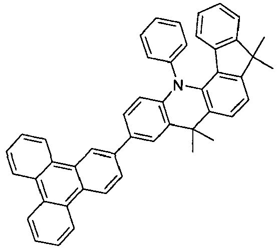 | |
| (135) | |
| 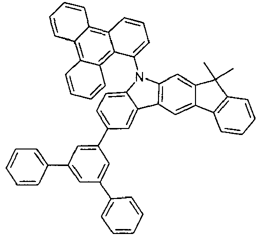 | 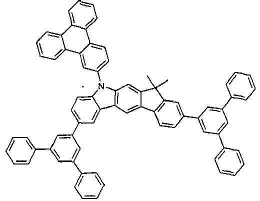 |
| (137) | (138) |
| 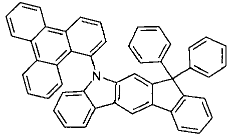 | 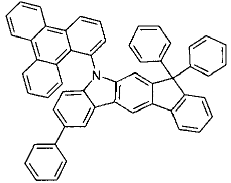 |
| (139) | (140) |
| 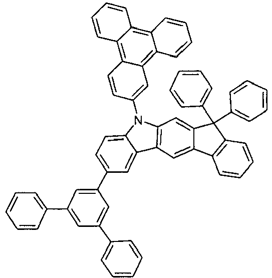 | 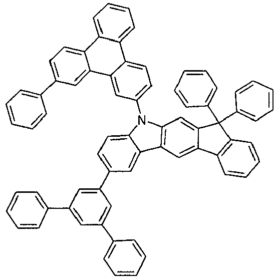 |
| (141) | (142) |
| 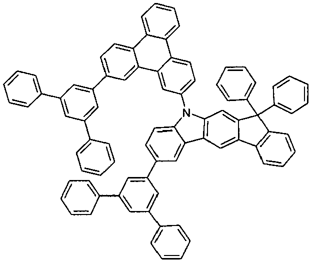 | 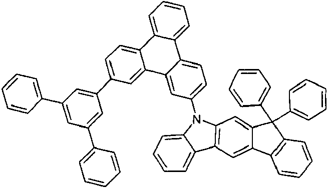 |
| (143) | (144) |
| 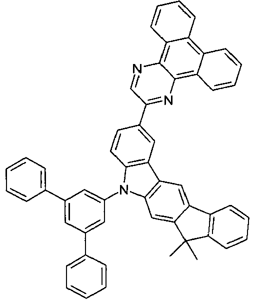 | 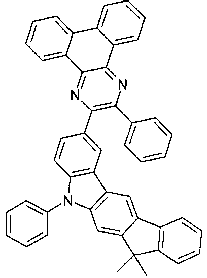 |
| (145) | (146) |
| | 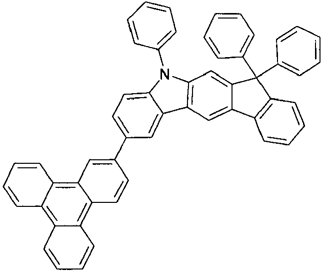 |
| | (148) |
| 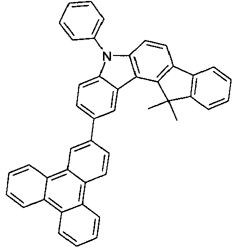 | 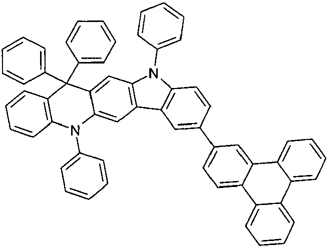 |
| (149) | (150) |
| 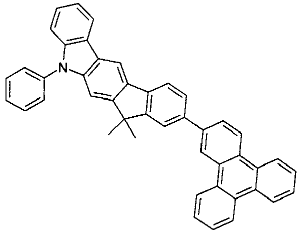 | |
| (151) | |
| 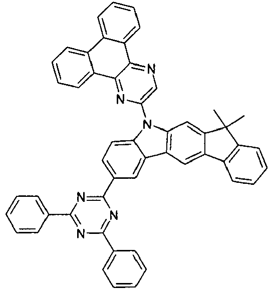 | 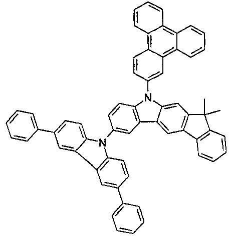 |
| (153) | (154) |
| | 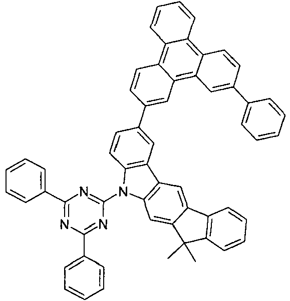 |
| | (156) |
| 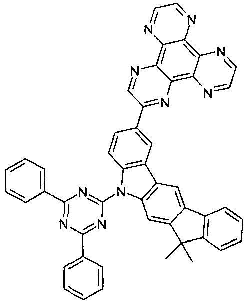 | |
| (157) | |
| 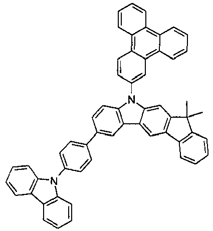 | 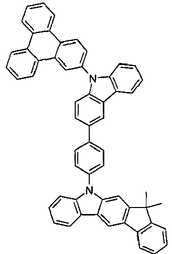 |
| (171) | (172) |
| 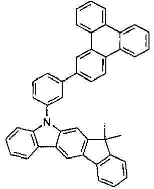 | 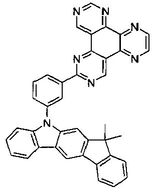 |
| (173) | (174) |
| 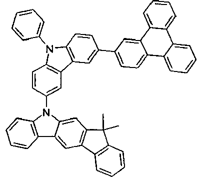 | 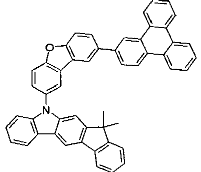 |
| (175) | (176) |
| 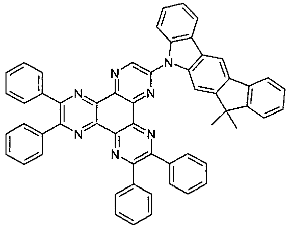 | |
| (177) | |
| 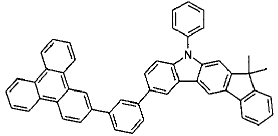 | 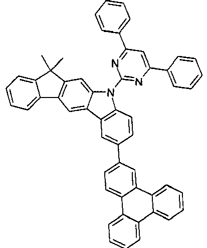 |
| (179) | (180) |
| 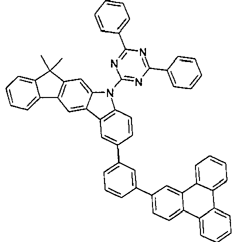 | 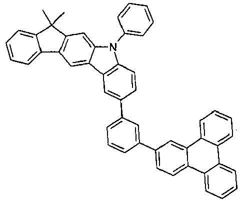 |
| (181) | (182) |
| 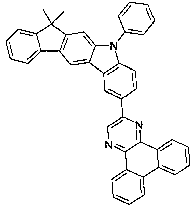 | 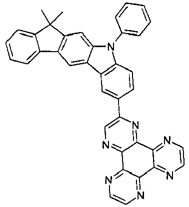 |
| (183) | (184) |
| 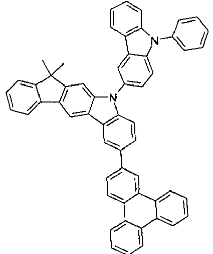 | 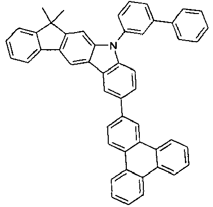 |
| (185) | (186) |
| 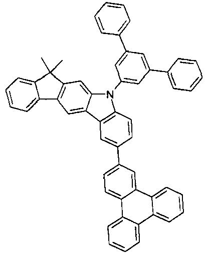 | 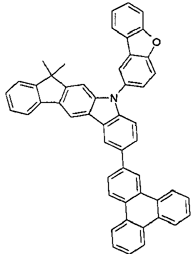 |
| (187) | (188) |
| 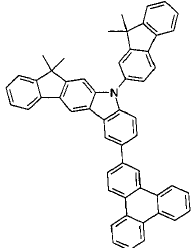 | 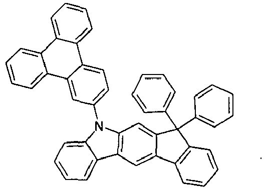 |
| (189) | (190) |
| 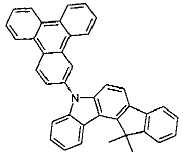 | 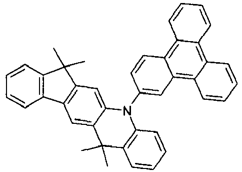 |
| (191) | (192) |
| 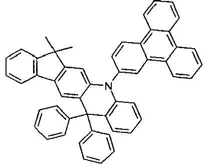 | |
| (193) | |

Die erfindungsgemäßen Verbindungen können nach dem Fachmann bekannten Syntheseschritten, wie z. B. Bromierung, Suzuki-Kupplung, Hartwig-Buchwald-Kupplung, etc., dargestellt werden. Die Synthese einer Verbindung der Formel (1), Formel (2), Formel (3) oder Formel (4), in der die Gruppe der Formel (5) an das Stickstoffatom gebunden ist, ist schematisch in Schema 1 dargestellt, wobei die Einführung der Triphenylengruppe bevorzugt über eine Hartwig-Buchwald-Kupplung unter Palladiumkatalyse erfolgt.

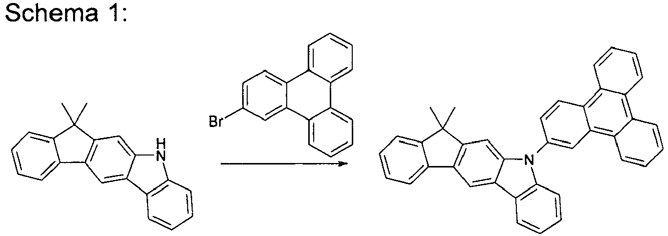

Ganz analog lassen sich substituierte Verbindungen herstellen, ebenso wie Verbindungen, die statt des Kohlenstoffatoms andere Brückenatome aufweisen oder Verbindungen, die zwischen dem Indenocarbazol und dem Triphenylen eine Gruppe Ar¹ aufweisen. Weitere Substituenten können auch durch nachträgliche Bromierung, gefolgt von einer Kupplungsreaktion, beispielsweise einer Suzuki-Kupplung oder Hartwig-Buchwald-Kupplung, eingeführt werden.

Ist die Gruppe der Formel (5) nicht am Stickstoffatom, sondern am Grundkörper des Indenocarbazols gebunden, eignet sich beispielsweise eine Suzuki-Kupplung zur Einführung dieser Gruppe, wie in Schema 2 dargestellt.

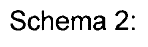

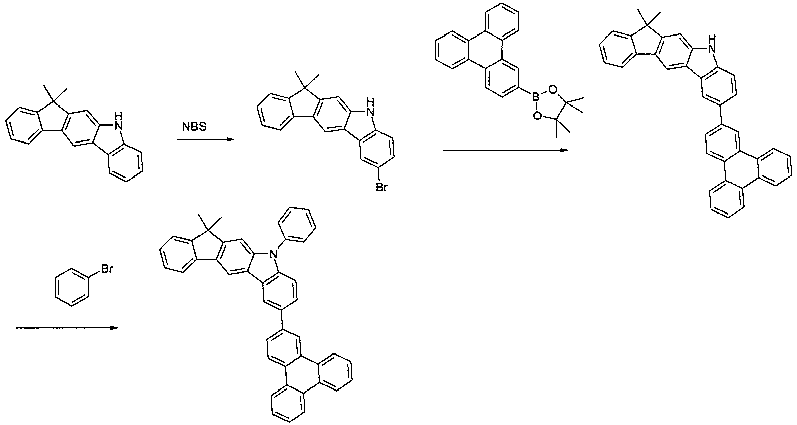

Ganz analog lassen sich substituierte Verbindungen herstellen, ebenso wie Verbindungen, die statt des Kohlenstoffatoms oder des Stickstoffatoms andere Brückenatome aufweisen oder Verbindungen, die zwischen dem Indenocarbazol und dem Triphenylen eine Gruppe Ar¹ aufweisen.

Ein weiterer Gegenstand der Erfindung ist ein Verfahren zur Herstellung einer erfindungsgemäßen Verbindung, gekennzeichnet durch die Schritte:
a) Synthese des Grundgerüsts der Formel (1), (2), (3) oder (4), das noch keine Gruppe gemäß Formel (5) aufweist; und
b) Einführung der Gruppe gemäß Formel (5) durch eine metallkatalysierte Kupplungsreaktion.

Als metallkatalysierte Kupplungsreaktion eignen sich beispielsweise Kupplungen gemäß Hartwig-Buchwald, Suzuki, Negishi, Kumada, Stille und andere Kupplungsreaktionen.

Ein weiterer Gegenstand der vorliegenden Erfindung sind Mischungen enthaltend mindestens eine erfindungsgemäße Verbindung und mindestens eine weitere Verbindung. Die weitere Verbindung kann beispielsweise ein fluoreszierender oder phosphoreszierender Dotand sein, wenn die erfindungsgemäße Verbindung als Matrixmaterial verwendet wird, insbesondere ein phosphoreszierender Dotand. Geeignete Dotanden sind unten im Zusammenhang mit den organischen Elektrolumineszenzvorrichtungen aufgeführt und sind auch für die erfindungsgemäßen Mischungen bevorzugt.

Für die Verarbeitung aus Lösung bzw. aus flüssiger Phase, beispielsweise durch Spin-Coating oder durch Druckverfahren, sind Lösungen bzw. Formulierungen der erfindungsgemäßen Verbindungen bzw. Mischungen erforderlich. Es kann bevorzugt sein, Mischungen aus zwei oder mehr Lösemitteln zu verwenden. Geeignete und bevorzugte Lösemittel sind beispielsweise Toluol, Anisol, o-, m- oder p-Xylol, Methylbenzoat, Dimethylanisol, Mesitylen, Tetralin, Veratrol, THF, Methyl-THF, THP, Chlorbenzol, Dioxan oder Mischungen dieser Lösemittel.

Ein weiterer Gegenstand der vorliegenden Erfindung ist daher eine Formulierung, insbesondere eine Lösung, eine Suspension oder eine Miniemulsion, enthaltend mindestens eine erfindungsgemäße Verbindung oder Mischung und ein oder mehrere Lösemittel, insbesondere organische Lösemittel. Wie solche Lösungen hergestellt werden können, ist dem Fachmann bekannt und beispielsweise in WO 2002/072714, WO 2003/019694 und der darin zitierten Literatur beschrieben.

Die erfindungsgemäßen Verbindungen und Mischungen eignen sich für die Verwendung in einer elektronischen Vorrichtung. Dabei wird unter einer elektronischen Vorrichtung eine Vorrichtung verstanden, welche mindestens eine Schicht enthält, die mindestens eine organische Verbindung enthält. Das Bauteil kann dabei aber auch anorganische Materialien enthalten oder auch Schichten, welche vollständig aus anorganischen Materialien aufgebaut sind.

Ein weiterer Gegenstand der vorliegenden Erfindung ist daher die Verwendung der erfindungsgemäßen Verbindungen oder Mischungen in einer elektronischen Vorrichtung, insbesondere in einer organischen Elektrolumineszenzvorrichtung.

Nochmals ein weiterer Gegenstand der vorliegenden Erfindung ist eine elektronische Vorrichtung enthaltend mindestens eine der oben ausgeführten erfindungsgemäßen Verbindungen oder Mischungen. Dabei gelten die oben für die Verbindung ausgeführten Bevorzugungen auch für die elektronischen Vorrichtungen.

Die elektronische Vorrichtung ist bevorzugt ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen farbstoff-sensibilisierten Solarzellen, organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices" (D. M. Koller et al., Nature Photonics 2008, 1-4), bevorzugt organischen Elektrolumineszenzvorrichtungen (OLEDs, PLEDs), insbesondere phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Zwischenschichten (Interlayer) eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen (für den prinzipiellen Aufbau siehe z. B. WO 2005/011013). Es kann sich dabei um fluoreszierende oder um phosphoreszierende Emissionsschichten handeln oder um Hybrid-Systeme, bei denen fluoreszierende und phosphoreszierende Emissionsschichten miteinander kombiniert werden.

Die erfindungsgemäße Verbindung gemäß den oben aufgeführten Ausführungsformen kann dabei in unterschiedlichen Schichten eingesetzt werden, je nach genauer Struktur. Bevorzugt ist eine organische Elektrolumineszenzvorrichtung, enthaltend eine Verbindung gemäß Formel (1), Formel (2), Formel (3), Formel (4) oder gemäß den bevorzugten Ausführungsformen als Matrixmaterial für fluoreszierende oder phosphoreszierende Emitter, insbesondere für phosphoreszierende Emitter, und/oder in einer Lochblockierschicht und/oder in einer Elektronentransportschicht und/oder in einer elektronenblockierenden bzw. exzitonenblockierenden Schicht und/oder in einer Lochtransportschicht, je nach genauer Substitution. Dabei gelten die oben aufgeführten bevorzugten Ausführungsformen auch für die Verwendung der Materialien in organischen elektronischen Vorrichtungen.

In einer bevorzugten Ausführungsform der Erfindung wird die Verbindung gemäß Formel (1), Formel (2), Formel (3), Formel (4) oder gemäß den bevorzugten Ausführungsformen als Matrixmaterial für eine fluoreszierende oder phosphoreszierende Verbindung, insbesondere für eine phosphoreszierende Verbindung, in einer emittierenden Schicht eingesetzt. Dabei kann die organische Elektrolumineszenzvorrichtung eine emittierende Schicht enthalten, oder sie kann mehrere emittierende Schichten enthalten, wobei mindestens eine emittierende Schicht mindestens eine erfindungsgemäße Verbindung als Matrixmaterial enthält.

Wenn die Verbindung gemäß Formel (1), Formel (2), Formel (3), Formel (4) oder gemäß den bevorzugten Ausführungsformen als Matrixmaterial für eine emittierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit höherer Spinmultiplizität verstanden, also einem Spinzustand > 1, insbesondere aus einem angeregten Triplettzustand. Im Sinne dieser Anmeldung sollen alle lumineszierenden Übergangsmetallkomplexe und lumineszierenden Lanthanidkomplexe, insbesondere alle Iridium-, Platin- und Kupferkomplexe als phosphoreszierende Verbindungen angesehen werden.

Die Mischung aus der Verbindung gemäß Formel (1), Formel (2), Formel (3), Formel (4) oder gemäß den bevorzugten Ausführungsformen und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der Verbindung gemäß Formel (1), Formel (2), Formel (3), Formel (4) oder gemäß den bevorzugten Ausführungsformen bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial.

Eine weitere bevorzugte Ausführungsform der vorliegenden Erfindung ist der Einsatz der Verbindung gemäß Formel (1), Formel (2), Formel (3), Formel (4) oder gemäß den bevorzugten Ausführungsformen als Matrixmaterial für einen phosphoreszierenden Emitter in Kombination mit einem weiteren Matrixmaterial. Besonders geeignete Matrixmaterialien, welche in Kombination mit den Verbindungen gemäß Formel (1), Formel (2), Formel (3), Formel (4) oder gemäß den bevorzugten Ausführungsformen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109 und WO 2011/000455, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, überbrückte Carbazol-Derivate, z. B. gemäß US 2009/0136779, WO 2010/050778, WO 2011/042107, WO 2011/088877 oder gemäß der nicht offen gelegten Anmeldung EP 11003232.3, Triphenylenderivate, z. B. gemäß der nicht offen gelegten DE 102010048608.6, oder Lactame, z. B. gemäß WO 2011/116865 und der nicht offen gelegten Anmeldung DE 102010019306.2. Ebenso kann ein weiterer phosphoreszierender Emitter, welcher kürzerwellig als der eigentliche Emitter emittiert, als Co-Host in der Mischung vorhanden sein.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten. Im Sinne der vorliegenden Erfindung werden alle lumineszierenden Verbindungen, die die oben genannten Metalle enthalten, als phosphoreszierende Verbindungen angesehen.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626 und WO 2011/066898 entnommen werden. Weiterhin eignen sich die Komplexe gemäß den nicht offen gelegten Anmeldungen EP 10006208.2 und DE 102010027317.1. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/ oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

In einer weiteren bevorzugten Ausführungsform der Erfindung wird die Verbindung gemäß Formel (1), Formel (2), Formel (3), Formel (4) bzw. gemäß den bevorzugten Ausführungsformen als Elektronentransportmaterial in einer Elektronentransport- oder Elektroneninjektionsschicht eingesetzt.

In nochmals einer weiteren bevorzugten Ausführungsform der Erfindung wird die Verbindung gemäß Formel (1), Formel (2), Formel (3), Formel (4) bzw. gemäß den bevorzugten Ausführungsformen in einer Lochblockierschicht eingesetzt.

Es ist weiterhin möglich, die Verbindung gemäß Formel (1), Formel (2), Formel (3), Formel (4) bzw. gemäß den bevorzugten Ausführungsformen sowohl in einer Lochblockierschicht bzw. Elektronentransportschicht als auch als Matrix in einer emittierenden Schicht zu verwenden.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (1), Formel (2), Formel (3), Formel (4) bzw. gemäß den bevorzugten Ausführungsformen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer oder höher ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Ink-Jet Druck (Tintenstrahldruck), LITI (Light Induced Thermal Imaging, Thermotransferdruck), Siebdruck, Flexodruck, Offsetdruck oder Nozzle-Printing hergestellt werden. Hierfür sind lösliche Verbindungen nötig, welche beispielsweise durch geeignete Substitution erhalten werden. Diese Verfahren eignen sich insbesondere auch für Oligomere, Dendrimere und Polymere.

Weiterhin sind Hybridverfahren möglich, bei denen beispielsweise eine oder mehrere Schichten aus Lösung aufgebracht werden und eine oder mehrere weitere Schichten aufgedampft werden. So ist es beispielsweise möglich, die emittierende Schicht aus Lösung aufzubringen und die Elektronentransportschicht aufzudampfen.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen weisen bei Verwendung in organischen Elektrolumineszenzvorrichtungen folgende überraschende Vorteile gegenüber dem Stand der Technik auf:
1. Die Leistungseffizienz entsprechender Vorrichtungen wird höher im Vergleich zu Systemen gemäß dem Stand der Technik.
2. Die Stabilität entsprechender Vorrichtungen wird höher im Vergleich zu Systemen gemäß dem Stand der Technik, was sich vor allem in einer deutlich höheren Lebensdauer zeigt.
3. Die erfindungsgemäßen organische Elektroluminszenzvorrichtungen weisen gleichzeitig eine verringerte Betriebsspannung auf.
4. Die erfindungsgemäßen organischen Elektrolumineszenzvorrichtungen weisen eine sehr hohe Effizienz auf. Die verbesserte Effizienz ist möglicherweise auf eine verbesserte Elektroneninjektion aus der Elektronentransportschicht in die emittierende Schicht zurückzuführen.

Die Erfindung wird nun durch die nachfolgenden Beispiele näher erläutert, ohne sie dadurch einschränken zu wollen.

### Beispiele

Die nachfolgenden Synthesen werden, sofern nicht anders angegeben, unter einer Schutzgasatmosphäre in getrockneten Lösungsmitteln durchgeführt. Die Lösungsmittel und Reagenzien können z. B. von Sigma-ALDRICH bzw. ABCR bezogen werden. Zu den literaturbekannten Verbindungen sind jeweils auch die entsprechenden CAS-Nummern angegeben.

### Beispiel 1: Synthese von Verbindung B1

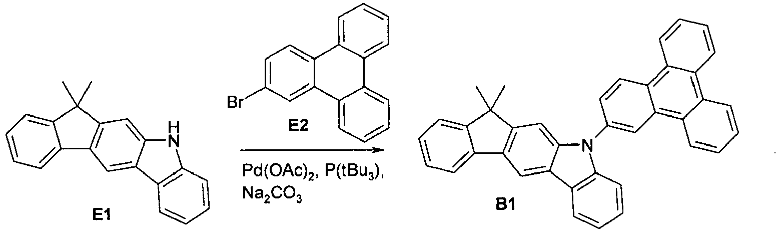

### B1: 12,12-Dimethyl-10-triphenylen-2-yl-10,12-dihydro-10-azaindeno[2,1-b]fluoren

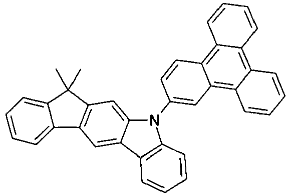

48 g (0.17 mol) 12,12-Dimethyl-10,12-dihydro-10-aza-indeno[2,1-b]fluoren (WO 2010/136109) und 45 g (0.16 mmol) 2-Bromotriphenylen (CAS 19111-87-6) werden in 2.5 L Xylol vorgelegt und entgast. Nach Zugabe von 50 g (0.52 mol) Natrium-*tert*-butanolat wird für 15 Minuten gerührt und anschließend mit 10 ml (10 mmol) Tri-*tert*-Butylphosphin und 1.5 g (6.7 mmol) Palladiumacetat versetzt. Der Ansatz wird für 40 h unter Rückfluss erhitzt. Nach beendeter Reaktion wird das Reaktionsgemisch mit Wasser gewaschen, die wässrige Phase mit Toluol extrahiert und die vereinten organischen Phasen über Natriumsulfat getrocknet. Das Lösungsmittel wird am Rotationsverdampfer entfernt und der Rückstand mit Toluol heiß extrahiert. Nach Sublimation werden 69 g (0.14 mol, 80%) des Produkts mit einer HPLC-Reinheit >99.9% erhalten.

Analog werden folgende Verbindungen dargestellt:

| **Bsp.** | **Edukt1** | **Edukt 2** | **Produkt** | **Ausbeute** |
|---|---|---|---|---|
| **B2** | 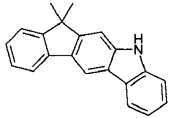 | 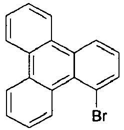 | 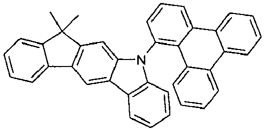 | 76 % |
| | WO 2010/136109 | CAS 19111-87-6 | | |
| **B3** | 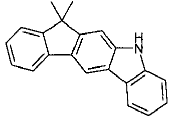 | 0.5 Equivalente: | 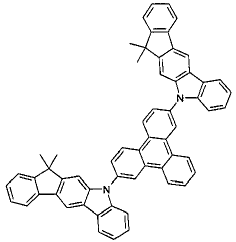 | 71 % |
| | | 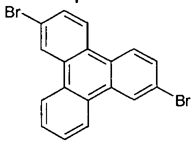 | | |
| | WO 2010/136109 | CAS 888041-37-0 | | |
| **B4** | 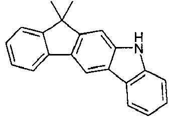 | 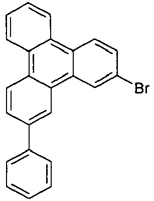 | 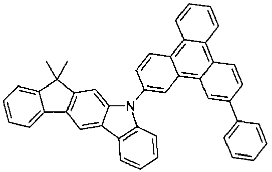 | 85 % |
| | WO 2010/136109 | CAS 24253-52-9 | | |
| **B5** | 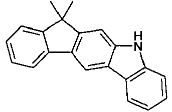 | 0.5 Equivalente: | 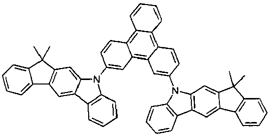 | 78 % |
| | | 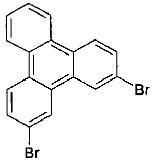 | | |
| | WO2010/136109 d | CAS 24253-51-8 | | |
| **B6** | 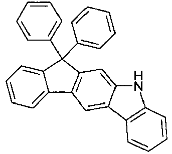 | 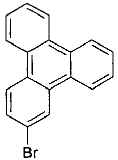 | 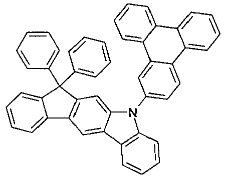 | 91 % |
| | WO 2010/136109 | CAS 19111-87-6 | | |
| **B7** | 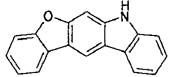 | 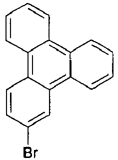 | 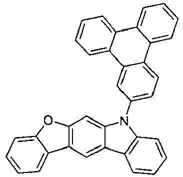 | 85 % |
| | CAS 1246308-83-7 | CAS 19111-87-6 | nicht erfindungsgemäß | |
| **B8** | 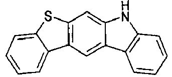 | 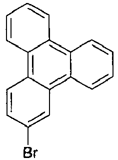 | 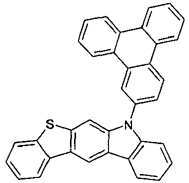 | 87 % |
| | CAS 1255309-04-6 | CAS 19111-87-6 | nicht erfindungsgemäß | |
| **B9** | 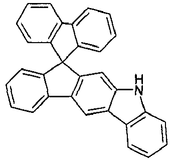 | 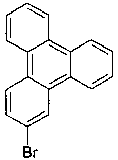 | 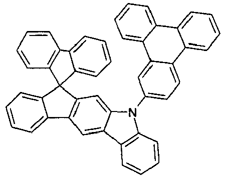 | 75 % |
| | WO 2010/136109 | CAS 19111-87-6 | nicht erfindungsgemäß | |
| **B10** | 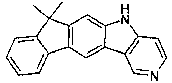 | 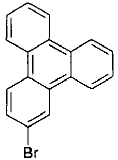 | 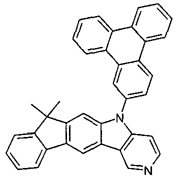 | 88 % |
| | WO2010/136109 | CAS 19111-87-6 | nicht erfindungsgemäß | |
| **B11** | 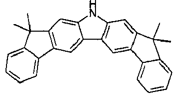 | 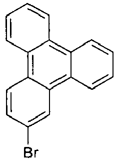 | 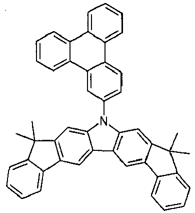 | 92 % |
| | WO2010/136109 | CAS 19111-876 | nicht erfindungsgemäß | |
| **B12** | 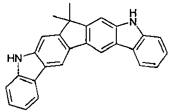 | 2 Equivalente: | 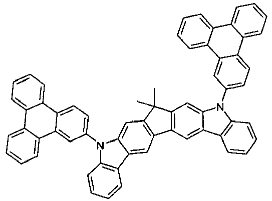 | 73 % |
| | | 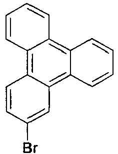 | | |
| | WO2010/136109 | CAS 19111-87-6 | nicht erfindungsgemäß | |
| **B13** | 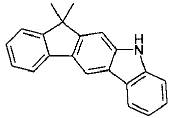 | 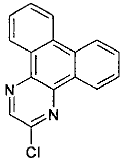 | 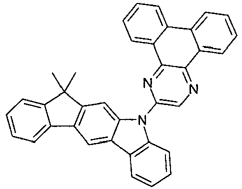 | 84 % |
| | WO 2010/136109 | CAS 1202564-31-5 | | |
| **B14** | 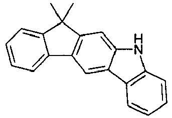 | 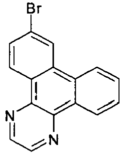 | 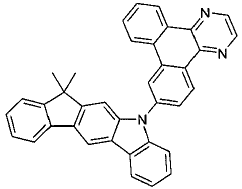 | 89 % |
| | WO 2010/136109 | CAS 1182724-82-8 | | |

### Beispiel 15: Synthese von Verbindung B15

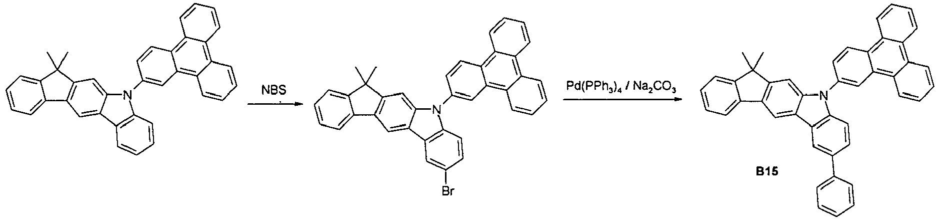

### Schritt 1: 7-Brom-12,12-dimethyl-10-triphenylen-2-yl-10,12-dihydro-10-aza-indeno[2,1-b]fluoren

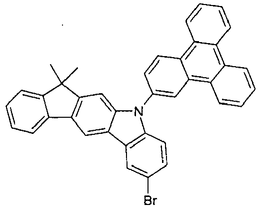

67.0 g (114 mmol) 12,12-Dimethyl-10-triphenylen-2-yl-10,12-dihydro-10-aza-indeno[2,1-b]fluoren werden in 500 mL Acetonitril vorgelegt. Anschließend tropft man unter Lichtausschluss bei -15 °C eine Lösung aus 20.3 g (114 mmol) NBS in 250 ml CH₃CN zu, lässt auf Raumtemperatur kommen und rührt 4 h weiter bei dieser Temperatur. Anschließend wird die Mischung mit 75 mL Wasser versetzt und mit CH₂Cl₂ extrahiert. Die organische Phase wird über MgSO₄ getrocknet und die Lösungsmittel im Vakuum entfernt. Das Produkt wird mit Hexan heiß ausgerührt und abgesaugt. Ausbeute: 38.7 g (66.1 mmol, 58 %), Reinheit nach ¹H-NMR ca. 96 %.

### Schritt 2: 12,12-Dimethyl-7-phenyl-10-triphenylen-2-yl-10,12-dihydro-10-aza-indeno-[2,1-b]fluoren (B15)

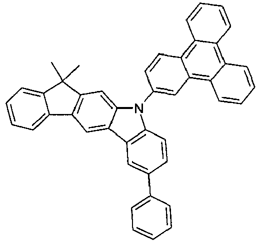

3.5 g (17 mmol) 4,4,5,5-Tetramethyl-2-phenyl-[1,3,2]dioxaborolan, 10 g (17 mmol) 7-Brom-12,12-dimethyl-10-triphenylen-2-yl-10,12-dihydro-10-aza-indeno[2,1-b]fluoren und 2.7 g Natriumcarbonat werden in 200 ml Dioxan, 200 ml Toluol und 100 ml Wasser suspendiert. Zu dieser Suspension werden 0.98 g (0.84 mmol) Pd(PPh₃)₄ gegeben. Die Reaktionsmischung wird 5 h unter Rückfluss erhitzt. Nach Erkalten wird der ausgefallene Feststoff abgesaugt, mit Wasser und Ethanol gewaschen und getrocknet. Der Rückstand wird mit Toluol heiß extrahiert und aus Toluol/Heptan umkristallisiert. Nach Sublimation werden 7.8 g (13 mmol, 79%) des Produktes mit einer HPLC Reinheit >99.9% erhalten.

Analog werden folgende Verbindungen daraestellt:

| Bsp. | Edukt | Produkt | Ausbeute |
|---|---|---|---|
| **B15** | 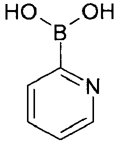 | 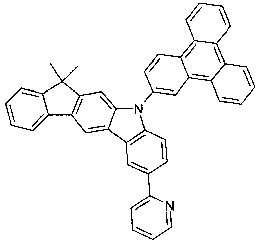 | 83 % |
| | CAS 197958-29-5 | | |
| **B16** | 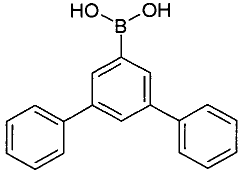 | 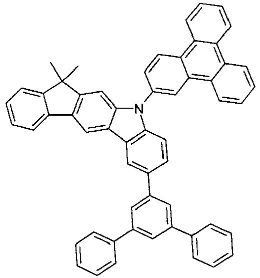 | 88 % |
| | CAS 128388-54-5 | | |
| **B17** | 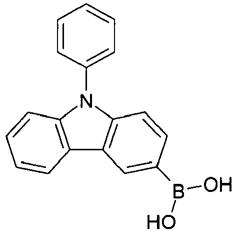 | 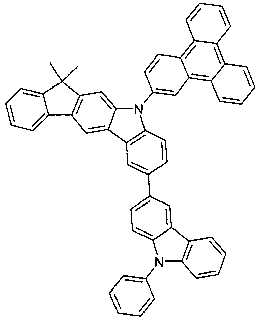 | 90% |
| | CAS 854952-58-2 | | |
| **B18** | 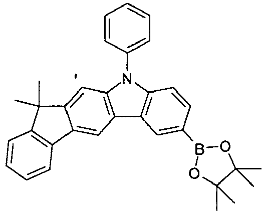 | 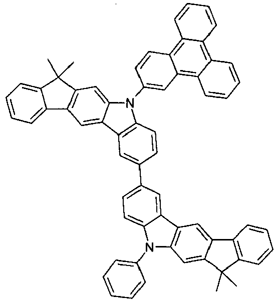 | 78 % |
| | **S1** | | |
| **B19** | 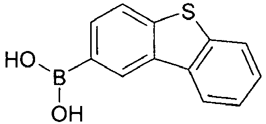 | 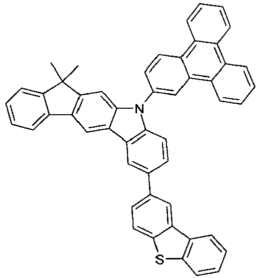 | 91 % |
| | CAS 668983-97-9 | | |

### Darstellung des Synthons S1 zu B18: 12,12-Dimethyl-10-phenyl-7-(4,4,5,5-tetramethyl-[1,3,2]dioxaborolan-2-yl)-10,12-dihydro-10-aza-indeno[2,1-b]fluoren:

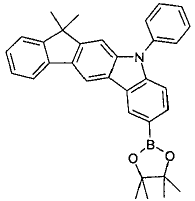

24 g (55 mmol) 7-Brom-12,12-dimethyl-10-phenyl-10,12-dihydro-10-aza-indeno[2,1-b]fluoren (WO2010136109), 15,5 g (60 mmol) Bis(pinacolato)-diboran und 16 g (160 mmol) Kaliumacetat werden in 200 ml Dioxan suspendiert. Zu dieser Suspension werden 1,3 g (1,7 mmol) 1,1-Bis(diphenylphosphino)ferrocen-dichloropalladium(II) Komplex mit Dichlormethan gegeben. Die Reaktionsmischung wird 7 h unter Rückfluss erhitzt. Nach Erkalten wird die organische Phase abgetrennt, dreimal mit je 100 mL Wasser gewaschen und anschließend zur Trockene eingeengt. Der Rückstand wird aus Toluol umkristallisiert (22 g, 45 mmol, 82%).

### B20: 7-Carbazol-9-yl-12,12-dimethyl-10-triphenylen-2-yl-10,12-dihydro-10-aza-indeno[2,1-b]fluoren

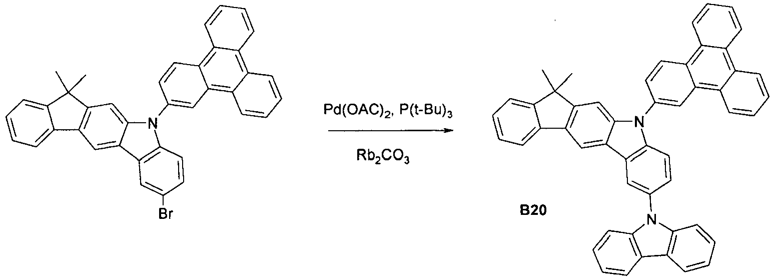

10 g (17 mmol) 7-Brom-12,12-dimethyl-10-triphenylen-2-yl-10,12-dihydro-10-aza-indeno[2,1-b]fluoren (Synthese: siehe oben), 9.2 g (19 mmol) Carbazol und 11.8 g Rb₂CO₃ werden in 125 mL p-Xylol suspendiert. Zu dieser Suspension werden 0.38 g (1.7 mmol) Pd(OAc)₂ und 5.1 ml einer 1M Tri-*tert*-butylphosphinlösung gegeben. Die Reaktionsmischung wird 16 h unter Rückfluss erhitzt. Nach Erkalten wird die organische Phase abgetrennt, dreimal mit 75 mL Wasser gewaschen und anschließend zur Trockene eingeengt. Der Rückstand wird mit Toluol heiß extrahiert, aus Toluol/Heptan umkristallisiert. Nach Sublimation werden 9.4 g (14 mmol, 82 %) des Produktes mit einer HPLC Reinheit > 99.9% erhalten.

Analog werden folgende Verbindungen dargestellt:

| Bsp. | Edukt | Produkt | Ausbeute |
|---|---|---|---|
| **B21** | 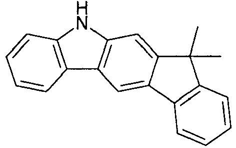 | 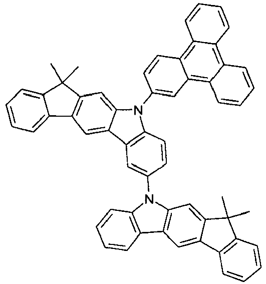 | 92% |
| **B22** | 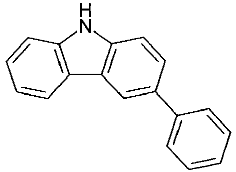 | 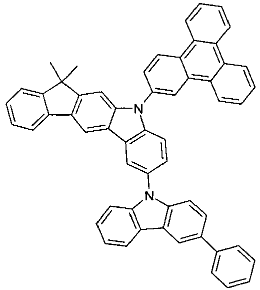 | 87 % |
| **B23** | 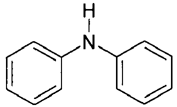 | 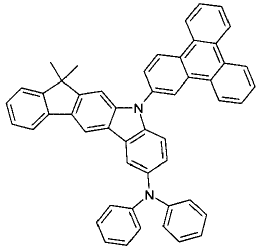 | 74 % |

### Synthese von B24:

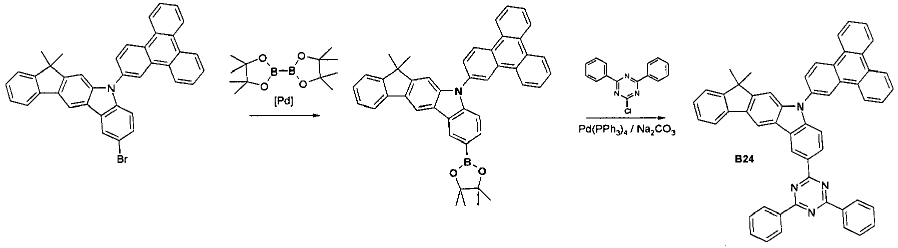

### Schritt 1: 12,12-Dimethyl-7-(4,4,5,5-tetramethyl-[1,3,2]dioxaborolan-2-yl)-10-triphenylen-2-yl-10,12-dihydro-10-aza-indeno[2,1-b]fluoren

13 g (22 mmol) 7-Bromo-12,12-dimethyl-10-triphenylen-2-yl-10,12-dihydro-10-aza-indeno[2,1-b]fluoren, 6.2 g (24 mmol) Bis(pinacolato)diboran und 6.3 g (64 mmol) Kaliumacetat werden in 75 ml Dioxan suspendiert. Zu dieser Suspension werden 0,53 g (0,66 mmol) 1,1-Bis(diphenylphosphino)-ferrocen-dichloropalladium(II) Komplex mit DCM gegeben. Die Reaktionsmischung wird 16 h unter Rückfluss erhitzt. Nach Erkalten wird die organische Phase abgetrennt, dreimal mit 50 mL Wasser gewaschen und anschließend zur Trockene eingeengt. Der Rückstand wird aus Toluol umkristallisiert (10.6 g, 17 mmol, 76%).

### B24: 7-(4,6-Diphenyl-[1,3,5]triazin-2-yl)-12,12-dimethyl-10-triphenylen-2-yl-10,12-dihydro-10-aza-indeno[2,1-b]fluoren

8.5 g (13.4 mmol) 12,12-Dimethyl-7-(4,4,5,5-tetramethyl-[1,3,2]dioxa-borolan-2-yl)-10-triphenylen-2-yl-10,12-dihydro-10-aza-indeno[2,1-b]-fluoren, 3.6 g (13.4 mmol) 2-Chlor-4,6-diphenyl-[1,3,5]triazin und 2.2 g Natriumcarbonat werden in 150 ml Dioxan, 150 ml Toluol und 75 ml Wasser suspendiert. Zu dieser Suspension werden 0.76 g (0.66 mmol) Pd(PPh₃)₄ gegeben. Die Reaktionsmischung wird 5 h unter Rückfluss erhitzt. Nach Erkalten wird der ausgefallene Feststoff abgesaugt, mit Wasser und Ethanol gewaschen und getrocknet. Der Rückstand wird mit Toluol heiß extrahiert und aus Toluol/Heptan umkristallisiert. Nach Sublimation werden 9.9 g (11 mmol, 82%) des Produkts mit einer HPLC Reinheit > 99.9% erhalten.

Analog werden folgende Verbindungen dargestellt:

| Bsp. | Edukt | Produkt | Ausbeute |
|---|---|---|---|
| **B25** | 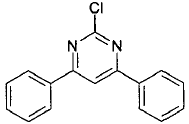 | 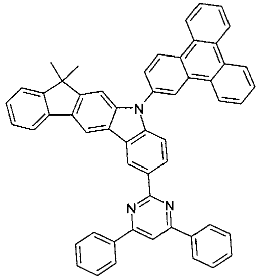 | 86% |
| **B26** | 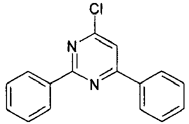 | 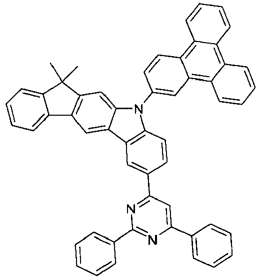 | 90 % |
| **B27** | 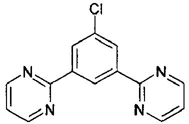 | 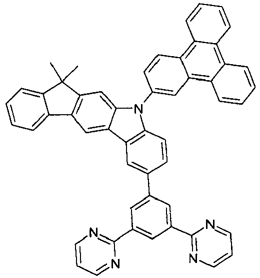 | 78 % |
| **B28** | 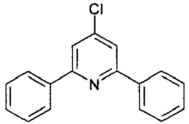 | 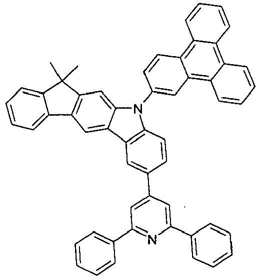 | 87 % |

### Synthese von B29:

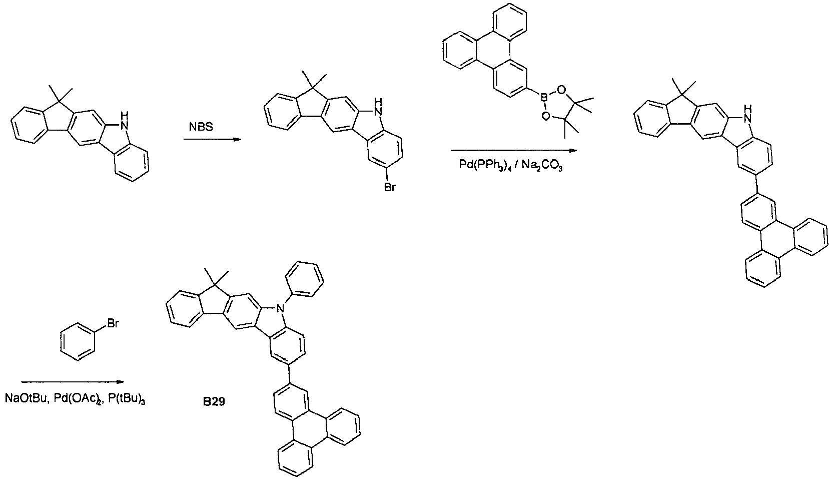

### Schritt 1: 7-Brom-12,12-dimethyl-10,12-dihydro-10-aza-indeno[2,1-b]fluoren

66.5 g (234.6 mmol) 12,12-Dimethyl-10,12-dihydro-10-aza-indeno[2,1-b]-fluoren werden in 1000 mL Acetonitril vorgelegt. Anschließend tropft man unter Lichtausschluss bei -15 °C eine Lösung aus 41.7 g (234.6 mmol) NBS in 500 ml CH₃CN zu, lässt auf Raumtemperatur kommen und rührt 4 h weiter bei dieser Temperatur. Anschließend wird die Mischung mit 150 mL Wasser versetzt und mit CH₂Cl₂ extrahiert. Die organische Phase wird über MgSO₄ getrocknet und die Lösungsmittel im Vakuum entfernt. Das Produkt wird mit Hexan heiß ausgerührt und abgesaugt. Ausbeute: 47.5 g (131 mmol), 56 % der Theorie, Reinheit nach ¹H-NMR ca. 97 %.

### Schritt 2: 12,12-Dimethyl-7-triphenylen-2-yl-10,12-dihydro-10-aza-indeno[2,1-b]fluoren

30.0 g (82.8 mmol) 7-Brom-12,12-dimethyl-10,12-dihydro-10-aza-indeno-[2,1-b]fluoren, 29.3 g (82.8 mmol) 4,4,5,5-Tetramethyl-2-triphenylen-2-yl-[1,3,2]dioxaborolan und 13.6 g Natriumcarbonat werden in 500 ml Dioxan, 500 ml Toluol und 250 ml Wasser suspendiert. Zu dieser Suspension werden 4.7 g (4.1 mmol) Pd(PPh₃)₄ gegeben. Die Reaktionsmischung wird 13 h unter Rückfluss erhitzt. Nach Erkalten wird der ausgefallene Feststoff abgesaugt, mit Wasser und Ethanol gewaschen und getrocknet. Der Rückstand wird mit Toluol heiß extrahiert und aus Toluol/Heptan umkristallisiert. Ausbeute: 37.6 g (11 mmol, 89%).

### Schritt 3: Synthese von 12,12-Dimethyl-10-phenyl-7-triphenylen-2-yl-10,12-dihydro-10-aza-indeno[2,1-b]fluoren (B29)

10.0 g (19.6 mmol) 12,12-Dimethyl-7-triphenylen-2-yl-10,12-dihydro-10-aza-indeno[2,1-b]fluoren und 4.6 g (29 mmol) Brombenzol werden in 250 ml Xylol vorgelegt und entgast. Nach Zugabe von 57.6 g (600 mmol) Natrium-*tert*-butanolat wird für 15 Minuten gerührt und anschließend mit 12 ml (12 mmol) Tri-*tert*-Butylphosphin und 1.7 g (7.7 mmol) Palladiumacetat versetzt. Der Ansatz wird für 28 h unter Rückfluss erhitzt. Nach beendeter Reaktion wird das Reaktionsgemisch mit Wasser gewaschen, die wässrige Phase mit Toluol extrahiert und die vereinten organischen Phasen über Natriumsulfat getrocknet. Das Lösungsmittel wird am Rotationsverdampfer entfernt und der Rückstand mit Toluol heißextrahiert. Es werden 10.7 g (18.3 mmol, 93%) des Produktes erhalten mit einer HPLC Reinheit > 99.9% erhalten.

Analog werden folgende Verbindungen dargestellt:

| Bsp. | Edukt | Produkt | Ausbeute |
|---|---|---|---|
| **B30** | 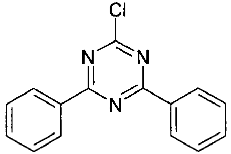 | 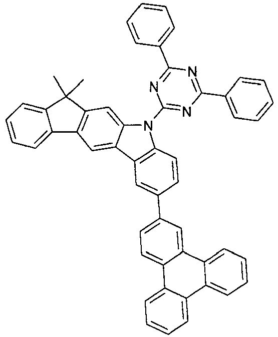 | 84 % |
| **B31** | 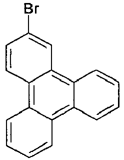 | 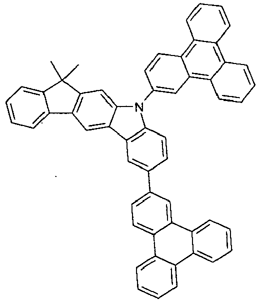 | 88 % |
| **B32** | 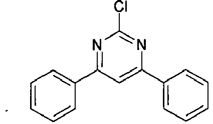 | 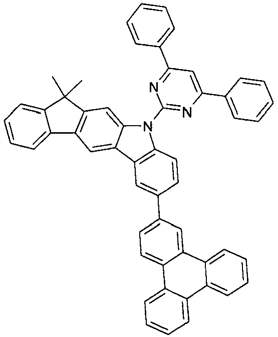 | 71 % |
| **B33** | 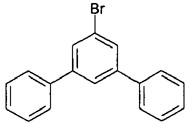 | 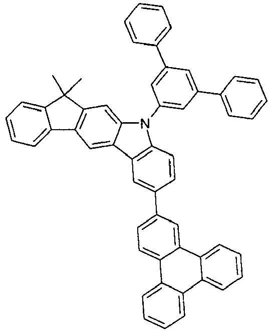 | 92 % |
| **B34** | 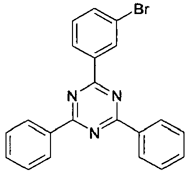 | 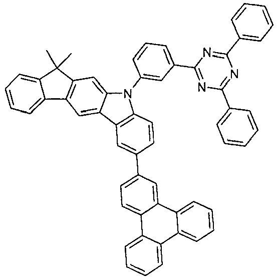 | 92 % |

### Beispiel: Herstellung der OLEDs

Die Herstellung von erfindungsgemäßen OLEDs sowie OLEDs nach dem Stand der Technik erfolgt nach einem allgemeinen Verfahren gemäß WO 2004/058911, das auf die hier beschriebenen Gegebenheiten (Schichtdickenvariation, Materialien) angepasst wird.

In den folgenden Beispielen V1 bis E24 (siehe Tabellen 1 und 2) werden die Daten verschiedener OLEDs vorgestellt. Glasplättchen, die mit strukturiertem ITO (Indium Zinn Oxid) der Dicke 50 nm beschichtet sind werden zur verbesserten Prozessierung mit 20 nm PEDOT:PSS beschichtet (Poly(3,4-ethylendioxythiophen)poly(styrolsulfonat), bezogen als CLEVIOS™ P VP Al 4083 von Heraeus Precious Metals GmbH Deutschland, aus wässriger Lösung aufgeschleudert). Diese beschichteten Glasplättchen bilden die Substrate, auf welche die OLEDs aufgebracht werden. Die OLEDs haben prinzipiell folgenden Schichtaufbau: Substrat / Lochtransportschicht (HTL) / Zwischenschicht (IL) / Elektronenblockerschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 3 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie IC1:IC3:TEG1 (70%:20%:10%) bedeutet hierbei, dass das Material IC1 in einem Volumenanteil von 70%, IC3 in einem Anteil von 20% und TEG1 in einem Anteil von 10% in der Schicht vorliegt.

Die OLEDs werden standardmäßig charakterisiert. Hierfür werden die Elektrolumineszenzspektren, die Stromeffizienz (gemessen in cd/A), die Leistungseffizienz (gemessen in Im/W und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe U1000 in Tabelle 2 bezeichnet die Spannung, die für eine Leuchtdichte von 1000 cd/m² benötigt wird. SE1000 und LE1000 bezeichnen die Strom- bzw. Leistungseffizienz, die bei 1000 cd/m² erreicht werden. EQE1000 schließlich bezeichnet die externe Quanteneffizienz bei einer Betriebsleuchtdichte von 1000 cd/m². Als Lebensdauer LD wird die Zeit definiert, nach der die Leuchtdichte bei Betrieb mit konstantem Strom von der Startleuchtdichte L0 auf einen gewissen Anteil L1 absinkt. Eine Angabe von L0 = 4000 cd/m² und L1 = 80% in Tabelle X2 bedeutet, dass die in Spalte LD angegebene Lebensdauer der Zeit entspricht, nach der die Anfangsleuchtdichte von 4000 cd/m² auf 3200 cd/m² absinkt.

Die Daten der verschiedenen OLEDs sind in Tabelle 2 zusammengefasst. Die Beispiele V1-V10 sind Vergleichsbeispiele gemäß dem Stand der Technik, die Beispiele E1-E24 zeigen Daten von OLEDs mit erfindungsgemäßen Materialien.

Im Folgenden werden einige der Beispiele näher erläutert, um die Vorteile der erfindungsgemäßen Verbindungen zu verdeutlichen. Es sei jedoch darauf hingewiesen, dass dies nur eine Auswahl der in Tabelle 2 gezeigten Daten darstellt. Wie sich der Tabelle entnehmen lässt, werden auch bei Verwendung der nicht näher ausgeführten erfindungsgemäßen Verbindungen deutliche Verbesserungen gegenüber dem Stand der Technik erzielt, teilweise in allen Parametern, in manchen Fällen ist aber nur eine Verbesserung von Effizienz oder Spannung oder Lebensdauer zu beobachten. Allerdings stellt bereits die Verbesserung einer der genannten Paramter einen signifikanten Fortschritt dar, weil verschiedene Anwendungen die Optimierung hinsichtlich unterschiedlicher Parameter erfordern.

### Verwendung von erfindungsgemäßen Verbindungen als Matrixmaterialien in phosphoreszierenden OLEDs

Vergleicht man das Material TPCbz1 mit dem erfindungsgemäßen Material B3 bei Einsatz als zweite Matrixkomponente in Kombination mit ST1, so zeigt sich, dass der Austausch von Carbazol gegen Indenocarbazol eine leichte Verbesserung der Leistungseffizienz, vor allem aber eine deutliche Verbesserung der Lebensdauer um etwa 70% bewirkt (Beispiele V7 und E6). Bei Austausch eines Biphenyls (Material BlC2) gegen Triphenylen (Material B3) als Verbindungsgruppe zwischen zwei Indenocarbazolen ergibt sich eine Verbesserung der Lebensdauer um etwa 50% (Beispiele V2 und E6).

Auch bei Verwendung nur eines Matrixmaterials in phosphoreszenten OLEDs hat die Substitution mit einem Triphenylen positiven Einfluss auf die Lebensdauer. So erhält man z. B. mit der Verbindung B34 eine Steigerung um fast 25% (Beispiele V4 und E20).

**Tabelle 1: Aufbau der OLEDs**

| Bsp. | HTL Dicke | IL Dicke | EBL Dicke | EML Dicke | HBL Dicke | ETL Dicke | EIL |
|---|---|---|---|---|---|---|---|
| V1 | SpA1 | HATCN | SpMA1 | IC1:BIC1:TEG1 | IC1 | ST1:LiQ (50%:50%) | --- |
| | 70nm | 5nm | 90nm | (60%:30%:10%) 30nm | 10nm | 30nm | |
| V2 | SpA1 | HATCN | BPA1 | ST1:BIC2:TEG1 | ST1 | ST1:LiQ (50%:50%) | --- |
| | 70nm | 5nm | 90nm | (30%:60%:10%) 30nm | 10nm | 30nm | |
| V3 | SpA1 | HATCN | SpMA1 | IC1:IC3:TEG1 | IC1 | ST1:LiQ (50%:50%) | --- |
| | 70nm | 5nm | 90nm | (70%:20%:10%) 30nm | 10nm | 30nm | |
| V4 | SpA1 | HATCN | SpMA1 | IC5:TER1 | --- | ST2:LiQ (50%:50%) | --- |
| | 90nm | 5nm | 130nm | (92%:8%) 40nm | | 40nm | |
| V5 | SpA1 | HATCN | SpMA1 | IC1:TEG1 | --- | ST1:LiQ (50%:50%) | --- |
| | 70nm | 5nm | 90nm | (90%:10%) 30nm | | 40nm | |
| V6 | SpA1 | HATCN | SpMA1 | ST1:IC4:TEG1 | IC1 | ST1:LiQ (50%:50%) | --- |
| | 70nm | 5nm | 90nm | (25%:60%:15%) 30nm | 10nm | 30nm | |
| V7 | SpA1 | HATCN | SpMA1 | ST1:TPCbz1:TEG1 | IC1 | ST1:LiQ (50%:50%) | --- |
| | 70nm | 5nm | 90nm | (25%:60%:15%) 30nm | 10nm | 30nm | |
| V8 | SpA1 | HATCN | SpMA1 | IC2:TEG1 | --- | ST1:LiQ (50%:50%) | --- |
| | 70nm | 5nm | 90nm | (90%:10%) 30nm | | 40nm | |
| V9 | SpA1 | HATCN | SpMA1 | IC5:TEG1 | --- | ST1:LiQ (50%:50%) | --- |
| | 70nm | 5nm | 90nm | (90%:10%) 30nm | | 40nm | |
| V10 | SpA1 | HATCN | SpMA1 | IC6:TEG1 | --- | ST1:LiQ (50%:50%) | --- |
| | 70nm | 5nm | 90nm | (90%:10%) 30nm | | 40nm | |
| E1 | SpA1 | HATCN | SpMA1 | ST1:B1:TEG1 | IC1 | ST1:LiQ (50%:50%) | --- |
| | 70nm | 5nm | 90nm | (25%:60%:15%) 30nm | 10nm | 30nm | |
| E2 | SpA1 | HATCN | SpMA1 | ST1:B2:TEG1 | IC1 | ST1:LiQ (50%:50%) | --- |
| | 70nm | 5nm | 90nm | (25%:60%:15%) 30nm | 10nm | 30nm | |
| E3 | SpA1 | HATCN | SpMA1 | ST1:B4:TEG1 | IC1 | ST1:LiQ (50%:50%) | --- |
| | 70nm | 5nm | 90nm | (25%:60%:15%) 30nm | 10nm | 30nm | |
| E4 | SpA1 | HATCN | SpMA1 | IC1:B17:TEG1 | IC1 | ST1:LiQ (50%:50%) | --- |
| | 70nm | 5nm | 90nm | (60%:35%:5%) 30nm | 10nm | 30nm | |
| E5 | SpA1 | HATCN | SpMA1 | IC1:B18:TEG1 | IC1 | ST1:LiQ (50%:50%) | --- |
| | 70nm | 5nm | 90nm | (60%:35%:5%) 30nm | 10nm | 30nm | |
| E6 | SpA1 | HATCN | BPA1 | ST1:B3:TEG1 | ST1 | ST1:LiQ (50%:50%) | --- |
| | 70nm | 5nm | 90nm | (30%:60%:10%) 30nm | 10nm | 30nm | |
| E7 | SpA1 | HATCN | SpMA1 | ST1:B29:TEG1 | IC1 | ST1:LiQ (50%:50%) | --- |
| | 70nm | 5nm | 90nm | (25%:60%:15%) 30nm | 10nm | 30nm | |
| E8* | SpA1 | HATCN | SpMA1 | IC1:B12:TEG1 | IC1 | ST1:LiQ (50%:50%) | --- |
| | 70nm | 5nm | 90nm | (70%:20%:10%) 30nm | 10nm | 30nm | |
| E9* | SpA1 | HATCN | SpMA1 | ST1:B10:TEG1 | IC1 | ST1:LiQ (50%:50%) | --- |
| | 70nm | 5nm | 90nm | (25%:60%:15%) 30nm | 10nm | 30nm | |
| E10* | SpA1 | HATCN | SpMA1 | ST1:B7:TEG1 | IC1 | ST1:LiQ (50%:50%) | --- |
| | 70nm | 5nm | 90nm | (25%:60%:15%) 30nm | 10nm | 30nm | |
| E11* | SpA1 | HATCN | SpMA1 | ST1:BB:TEG1 | IC1 | ST1:LiQ (50%:50%) | --- |
| | 70nm | 5nm | 90nm | (25%:60%:15%) 30nm | 10nm | 30nm | |
| E12 | SpA1 | HATCN | SpMA1 | IC1:B21:TEG1 | IC1 | ST1:LiQ (50%:50%) | --- |
| | 70nm | 5nm | 90nm | (30%:60%:10%) 30nm | 10nm | 30nm | |
| E13 | SpA1 | HATCN | SpMA1 | IC1:B20:TEG1 | IC1 | ST1:LiQ (50%:50%) | --- |
| | 70nm | 5nm | 90nm | (30%:60%:10%) 30nm | 10nm | 30nm | |
| E14 | SpA1 | HATCN | SpMA1 | IC1:B19:TEG1 | IC1 | ST1:LiQ (50%:50%) | --- |
| | 70nm | 5nm | 90nm | (30%:60%:10%) 30nm | 10nm | 30nm | |
| E15 | SpA1 | HATCN | SpMA1 | B24:TEG1 (90%:10%) | --- | ST1:LiQ (50%:50%) | --- |
| | 70nm | 5nm | 90nm | 30nm | | 40nm | |
| E16 | SpA1 | HATCN | SpMA1 | B25:TEG1 (90%:10%) | --- | ST1:LiQ (50%:50%) | --- |
| | 70nm | 5nm | 90nm | 30nm | | 40nm | |
| E17 | SpA1 | HATCN | SpMA1 | B30:TEG1 (90%:10%) | --- | ST1:LiQ (50%:50%) | --- |
| | 70nm | 5nm | 90nm | 30nm | | 40nm | |
| E18 | SpA1 | HATCN | SpMA1 | B32:TEG1 (90%:10%) | --- | ST1:LiQ (50%:50%) | --- |
| | 70nm | 5nm | 90nm | 30nm | | 40nm | |
| E19 | SpA1 | HATCN | SpMA1 | B34:TEG1 (90%:10%) | --- | ST1:LiQ (50%:50%) | --- |
| | 70nm | 5nm | 90nm | 30nm | | 40nm | |
| E20 | SpA1 | HATCN | SpMA1 | B34:TER1 | --- | ST2:LiQ (50%:50%) | --- |
| | 90nm | 5nm | 130nm | (92%:8%) 40nm | | 40nm | |
| E21 | SpA1 | HATCN | SpMA1 | B13:TER1 | --- | ST2:LiQ (50%:50%) | --- |
| | 90nm | 5nm | 130nm | (92%:8%) 40nm | | 40nm | |
| E22* | SpA1 | HATCN | SpMA1 | IC1:B11:TEG1 | IC1 | ST1:LiQ (50%:50%) | --- |
| | 70nm | 5nm | 90nm | (30%:60%:10%) 30nm | 10nm | 30nm | |
| E23 | SpA1 | HATCN | SpMA1 | ST1:IC4:TEG1 | B30 | ST1:LiQ (50%:50%) | --- |
| | 70nm | 5nm | 90nm | (25%:60%:15%) 30nm | 10nm | 30nm | |
| E24 | SpA1 | HATCN | SpMA1 | IC1:TEG1 | --- | B13 | LiQ 3nm |
| | 70nm | 5nm | 90nm | (90%:10%) 30nm | | 40 nm | |

| | | | | | | | |
|---|---|---|---|---|---|---|---|
| * nicht erfindungsgemäß | | | | | | | |

**Tabelle 2: Daten der OLEDs**

| Bsp. | U1000 (V) | SE1000 (cd/A) | LE1000 (Im/W) | EQE 1000 | CIE x/y bei 1000 cd/m² | L0 | L1 % | LD (h) |
|---|---|---|---|---|---|---|---|---|
| V1 | 3.6 | 51 | 44 | 14.1% | 0.34/0.62 | 10000 cd/m² | 70 | 240 |
| V2 | 3.8 | 51 | 43 | 14.3% | 0.33/0.63 | 10000 cd/m² | 70 | 210 |
| V3 | 3.2 | 54 | 54 | 15.1% | 0.33/0.62 | 10000 cd/m² | 80 | 190 |
| V4 | 4.5 | 11.2 | 7.8 | 12.1% | 0.67/0.33 | 4000 cd/m² | 80 | 410 |
| V5 | 3.5 | 57 | 52 | 16.2% | 0.34/0.62 | 10000 cd/m² | 70 | 160 |
| V6 | 3.5 | 56 | 50 | 15.7% | 0.34/0.62 | 10000 cd/m² | 70 | 350 |
| V7 | 3.8 | 50 | 42 | 13.9% | 0.33/0.63 | 10000 cd/m² | 70 | 190 |
| V8 | 3.5 | 59 | 52 | 16.4% | 0.33/0.62 | 10000 cd/m² | 70 | 160 |
| V9 | 3.3 | 53 | 51 | 14.8% | 0.33/0.61 | 10000 cd/m² | 70 | 150 |
| V10 | 3.6 | 57 | 49 | 15.8% | 0.33/0.62 | 10000 cd/m² | 70 | 150 |
| E1 | 3.5 | 60 | 54 | 16.7% | 0.34/0.62 | 10000 cd/m² | 70 | 420 |
| E2 | 3.5 | 57 | 52 | 16.1% | 0.35/0.62 | 10000 cd/m² | 70 | 410 |
| E3 | 3.6 | 56 | 50 | 15.8% | 0.34/0.62 | 10000 cd/m² | 70 | 430 |
| E4 | 3.4 | 60 | 55 | 16.7% | 0.33/0.63 | 10000 cd/m² | 70 | 450 |
| E5 | 3.6 | 56 | 48 | 15.5% | 0.33/0.62 | 10000 cd/m² | 70 | 330 |
| E6 | 3.7 | 53 | 45 | 14.7% | 0.33/0.62 | 10000 cd/m² | 70 | 320 |
| E7 | 3.6 | 57 | 51 | 16.0% | 0.34/0.63 | 10000 cd/m² | 70 | 370 |
| E8* | 3.3 | 61 | 57 | 16.9% | 0.33/0.62 | 10000 cd/m² | 80 | 230 |
| E9* | 3.5 | 51 | 49 | 14.3% | 0.33/0.63 | 10000 cd/m² | 70 | 370 |
| E10* | 3.6 | 57 | 50 | 15.8% | 0.32/0.62 | 10000 cd/m² | 70 | 370 |
| E11* | 3.6 | 55 | 48 | 15.2% | 0.34/0.62 | 10000 cd/m² | 70 | 400 |
| E12 | 3.5 | 50 | 44 | 13.9% | 0.33/0.62 | 10000 cd/m² | 70 | 380 |
| E13 | 3.5 | 55 | 50 | 15.3% | 0.33/0.62 | 10000 cd/m² | 70 | 390 |
| E14 | 3.4 | 60 | 55 | 16.7% | 0.33/0.63 | 10000 cd/m² | 70 | 360 |
| E15 | 3.5 | 60 | 54 | 16.5% | 0.33/0.63 | 10000 cd/m² | 70 | 130 |
| E16 | 3.5 | 59 | 52 | 16.3% | 0.33/0.62 | 10000 cd/m² | 70 | 140 |
| E17 | 3.6 | 56 | 49 | 15.5% | 0.32/0.62 | 10000 cd/m² | 70 | 190 |
| E18 | 3.5 | 61 | 55 | 17.0% | 0.33/0.62 | 10000 cd/m² | 70 | 140 |
| E19 | 3.4 | 54 | 50 | 15.2% | 0.33/0.62 | 10000 cd/m² | 70 | 200 |
| E20 | 4.6 | 10.7 | 7.3 | 11.6% | 0.67/0.33 | 4000 cd/m² | 80 | 510 |
| E21 | 4.2 | 11.8 | 8.7 | 12.8% | 0.67/0.33 | 4000 cd/m² | 80 | 380 |
| E22* | 3.6 | 59 | 51 | 16.4% | 0.34/0.62 | 10000 cd/m² | 70 | 350 |
| E23 | 3.5 | 55 | 49 | 15.4% | 0.34/0.62 | 10000 cd/m² | 70 | 380 |
| E24 | 3.7 | 56 | 49 | 15.7% | 0.33/0.63 | 10000 cd/m² | 70 | 190 |

| | | | | | | | | |
|---|---|---|---|---|---|---|---|---|
| * nicht erfindungsgemäß | | | | | | | | |

**Tabelle 3: Strukturformeln der Materialien für die OLEDs**

| | |
|---|---|
| 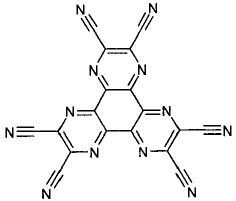 | 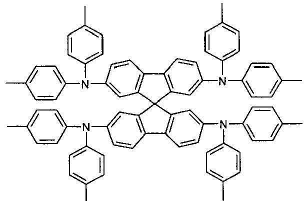 |
| HATCN | SpA1 |
| 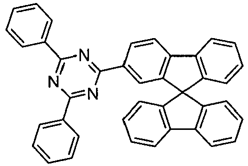 | 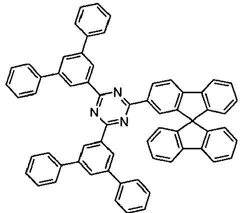 |
| ST1 | ST2 |
| 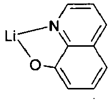 | 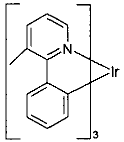 |
| LiQ | TEG1 |
| 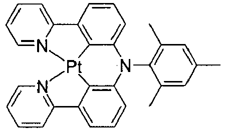 | 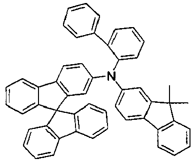 |
| TER1 | SpMA1 |
| 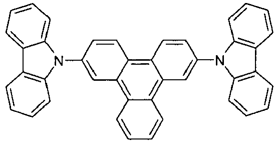 | 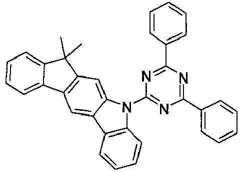 |
| TPCbz1 (Stand der Technik) | IC1 (Stand der Technik) |
| 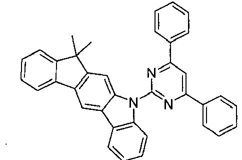 | 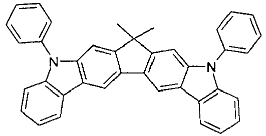 |
| IC2 (Stand der Technik) | IC3 (Stand der Technik) |
| 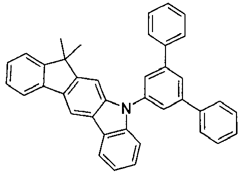 | 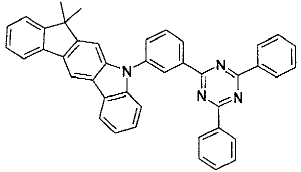 |
| IC4 (Stand der Technik) | IC5 (Stand der Technik) |
| 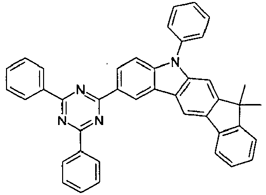 | 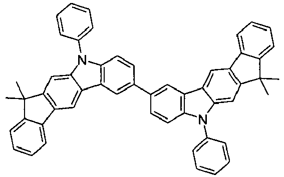 |
| IC6 (Stand der Technik) | BIC1 (Stand der Technik) |
| 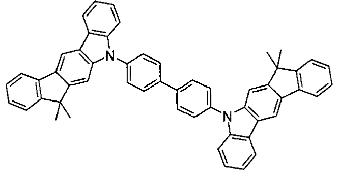 | |
| BIC2 (Stand der Technik) | |

## Patentansprüche

1. Verbindung gemäß Formel (1), 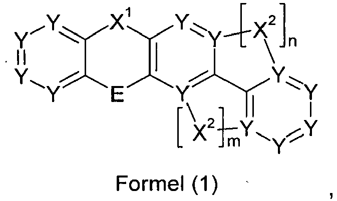 wobei für die verwendeten Symbole gilt:
Y ist C, wenn an diese Gruppe Y eine Gruppe X² gebunden ist, bzw. ist bei jedem Auftreten gleich oder verschieden CR, wenn an diese Gruppe Y keine Gruppe X² gebunden ist;
E ist eine Einfachbindung;
X¹ ist eine bivalente Brücke und steht für N(R¹);
X² ist eine bivalente Brücke und steht für C(R¹)₂;
oder Verbindung der Formel (2), Formel (3) oder Formel (4), 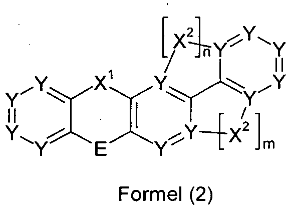 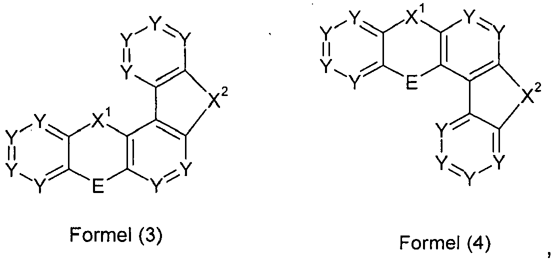 wobei für die verwendeten Symbole gilt:
Y ist C, wenn an diese Gruppe Y eine Gruppe X² gebunden ist, bzw. ist bei jedem Auftreten gleich oder verschieden CR oder N, wenn an diese Gruppe Y keine Gruppe X² gebunden ist;
E ist eine Einfachbindung oder eine bivalente Brücke, ausgewählt aus N(R¹), C(R¹)₂, O oder S;
X¹, X² ist bei jedem Auftreten gleich oder verschieden eine bivalente Brücke, ausgewählt aus N(R¹), O, C(R¹)₂ oder S; mit der Maßgabe, dass X¹ und X² nicht gleichzeitig für O stehen;
weiterhin gilt für die verwendeten Symbole und Indizes in Formeln (1), (2), (3) und (4):
R ist bei jedem Auftreten gleich oder verschieden H oder D oder die Reste R, die ungleich H oder D sind, sind ausgewählt aus der Gruppe bestehend aus N(Ar)₂, einer geradkettigen Alkylgruppe mit 1 bis 10 C-Atomen, einer verzweigten Alkylgruppe mit bis zu 10 C-Atomen, oder einem aromatischen oder heteroaromatischen Ringsystem mit 5 bis 40 Ringatomen, das durch ein oder mehrere Reste R² substituiert sein kann;
R¹ steht, wenn E, X¹ und/oder X² für NR¹ stehen, für ein aromatisches oder heteroaromatisches Ringsystem 6 bis 24 aromatischen Ringatomen, das durch einen oder mehrere Reste R² substituiert sein kann;
und steht, wenn E, X¹ und/oder X² für C(R¹)₂ stehen, für eine geradkettige Alkylgruppe mit 1 bis 20 C-Atomen oder eine verzweigte oder cyclische Alkylgruppe mit 3 bis 20 C-Atomen, die jeweils mit einem oder mehreren Resten R² substituiert sein kann, wobei ein oder mehrere H-Atome durch D, F oder CN ersetzt sein können, oder für ein aromatisches oder heteroaromatisches Ringsystem mit 5 bis 60 aromatischen Ringatomen, das durch einen oder mehrere Reste R² substituiert sein kann;
Ar ist bei jedem Auftreten gleich oder verschieden ein aromatisches oder heteroaromatisches Ringsystem, bevorzugt eine Aryl- oder Heteroarylgruppe mit 5 bis 40 Ringatomen, das mit einem oder mehreren Resten R³ substituiert sein kann;
R² ist bei jedem Auftreten gleich oder verschieden H, D, F, N(Ar)₂, CN, eine geradkettige Alkylgruppe mit 1 bis 40 C-Atomen oder eine verzweigte oder cyclische Alkylgruppe mit 3 bis 40 C-Atomen, wobei ein oder mehrere H-Atome durch D, F oder CN ersetzt sein können, oder ein aromatisches oder heteroaromatisches Ringsystem mit 5 bis 60 aromatischen Ringatomen, das jeweils durch einen oder mehrere Reste R³ substituiert sein kann;
R³ ist bei jedem Auftreten gleich oder verschieden H, D oder ein aliphatischer Kohlenwasserstoffrest mit 1 bis 20 C-Atomen oder eine Aryl- oder Heteroarylgruppe mit 5 bis 40 Ringatomen oder eine Kombination dieser Gruppen;
n, m sind gleich oder verschieden bei jedem Auftreten 0 oder 1, mit der Maßgabe, dass n + m = 1 oder 2 ist;
**dadurch gekennzeichnet, dass** in der Verbindung der Formel (1), (2), (3) bzw. (4) mindestens eine Gruppe R und/oder R¹ vorhanden ist, die für eine Gruppe der folgenden Formel (5) steht, 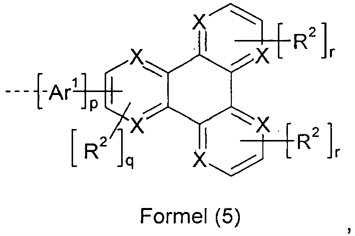 wobei die gestrichelte Bindung die Verknüpfung der Gruppe der Formel (5) andeutet, R² die oben genannten Bedeutungen aufweist und weiterhin gilt:
X ist C, wenn an dieses X die Gruppe Ar¹ bzw. das restliche Molekül gebunden ist, und ist bei jedem Auftreten gleich oder verschieden CR² oder N in den sonstigen Fällen;
Ar¹ ist ein bivalentes aromatisches oder heteroaromatisches Ringsystem mit 5 bis 40 aromatischen Ringatomen, welches durch einen oder mehrere Reste R² substituiert sein kann;
p ist bei jedem Auftreten gleich oder verschieden 0 oder 1;
q, r ist bei jedem Auftreten gleich oder verschieden 0, 1 oder 2.

2. Verbindung gemäß Formel (2), (3) oder (4) nach Anspruch 1, **dadurch gekennzeichnet, dass** X¹ für N(R¹) und X² für C(R¹)₂ steht und dass E gleichzeitig für eine Einfachbindung steht.

3. Verbindung nach Anspruch 1 oder 2, **dadurch gekennzeichnet, dass** Y für C steht, wenn an diese Gruppe Y eine Gruppe X² gebunden ist, oder gleich oder verschieden bei jedem Auftreten für CR steht, wenn an diese Gruppe Y keine Gruppe X² gebunden ist.

4. Verbindung nach einem oder mehreren der Ansprüche 1 bis 3, **dadurch gekennzeichnet, dass** X für CR² steht, bzw. dass X für C steht, wenn an dieses X die Gruppe Ar¹ bzw. das restliche Molekül gebunden ist.

5. Verbindung nach einem oder mehreren der Ansprüche 1 bis 4, **dadurch gekennzeichnet, dass** die Verbindung ausgewählt ist aus Verbindungen gemäß Formel (1a), (2a), (3a) oder (4a), 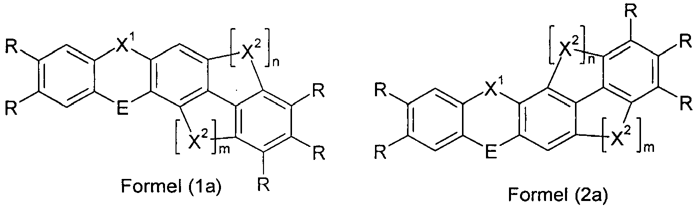 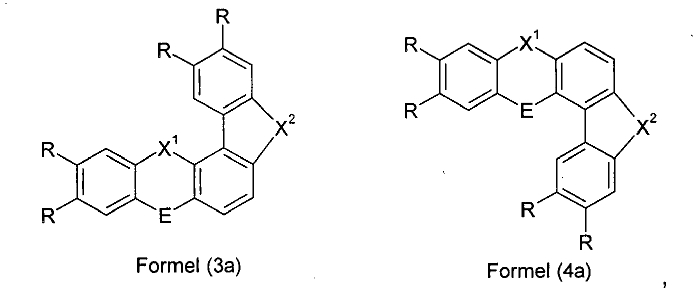 wobei die verwendeten Symbole und Indizes die in Anspruch 1 genannten Bedeutungen aufweisen und X¹ bevorzugt für NR¹ steht.

6. Verbindung nach einem oder mehreren der Ansprüche 1 bis 5, **dadurch gekennzeichnet, dass** die Verbindung ausgewählt aus Verbindungen gemäß Formel (1e): 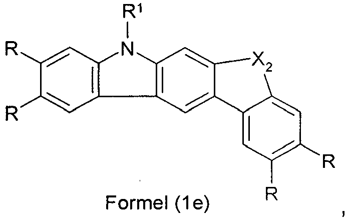 wobei die Symbole und Indizes die Anspruch 1 angegebenen Bedeutungen haben.

7. Verbindung nach einem oder mehreren der Ansprüche 1 bis 6, **dadurch gekennzeichnet, dass** die Gruppe gemäß Formel (5) eine Gruppe der Formel (5a) darstellt, 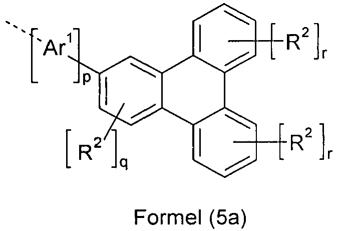 wobei die verwendeten Symbole und Indizes die in Anspruch 1 genannten Bedeutungen aufweisen.

8. Verbindung nach einem oder mehreren der Ansprüche 1 bis 7, **dadurch gekennzeichnet, dass** die Gruppe gemäß Formel (5) eine Gruppe der Formel (5b) oder (5c) darstellt, 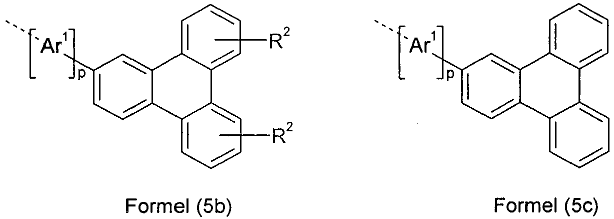 wobei die verwendeten Symbole und Indizes die in Anspruch 1 genannten Bedeutungen aufweisen.

9. Verfahren zur Herstellung einer Verbindung nach einem oder mehreren der Ansprüche 1 bis 8, **gekennzeichnet durch** die Schritte:
a) Synthese des Grundgerüsts der Formel (1), (2), (3) oder (4), das noch keine Gruppe gemäß Formel (5) aufweist; und
b) Einführung der Gruppe gemäß Formel (5) durch eine metallkatalysierte Kupplungreaktion.

10. Mischung, enthaltend mindestens eine Verbindung nach einem oder mehreren der Ansprüche 1 bis 8 und mindestens eine weitere Verbindung, insbesondere einen phosphoreszierenden Dotanden.

11. Formulierung, insbesondere eine Lösung, eine Suspension oder eine Miniemulsion, enthaltend mindestens eine Verbindung nach einem oder mehreren der Ansprüche 1 bis 8 oder eine Mischung nach Anspruch 10 und ein oder mehrere Lösemittel.

12. Verwendung einer Verbindung nach einem oder mehreren der Ansprüche 1 bis 8 oder einer Mischung nach Anspruch 10 in einer elektronischen Vorrichtung.

13. Elektronische Vorrichtung enthaltend mindestens eine Verbindung nach einem oder mehreren der Ansprüche 1 bis 8 oder eine Mischung nach Anspruch 10, insbesondere ausgewählt aus der Gruppe bestehend aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, organischen farbstoff-sensibilisierten Solarzellen, organischen optischen Detektoren, organischen Photorezeptoren, organischen Feld-Quench-Devices, lichtemittierenden elektrochemischen Zellen, organischen Laserdioden und "organic plasmon emitting devices".

14. Elektronische Vorrichtung nach Anspruch 13, **dadurch gekennzeichnet, dass** es sich um eine organische Elektrolumineszenzvorrichtung handelt und die Verbindung nach einem oder mehreren der Ansprüche 1 bis 8 als Matrixmaterial für fluoreszierende oder phosphoreszierende Emitter und/oder in einer Lochblockierschicht und/oder in einer Elektronentransportschicht und/oder in einer elektronenblockierenden bzw. exzitonenblockierenden Schicht und/oder in einer Lochtransportschicht eingesetzt wird.

## Claims

1. Compound of the formula (1), 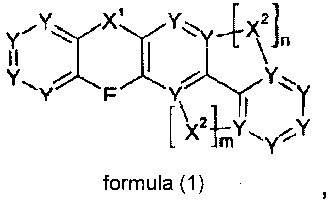 where the following applies to the symbols used:
Y is C if a group X² is bonded to this group Y, or is on each occurrence, identically or differently, CR if no group X² is bonded to this group Y;
E is a single bond;
X¹ is a divalent bridge and stands for N(R¹);
X² is a divalent bridge and stands for C(R¹)₂;
or compound of the formula (2), formula (3) or formula (4), 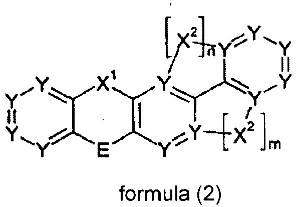 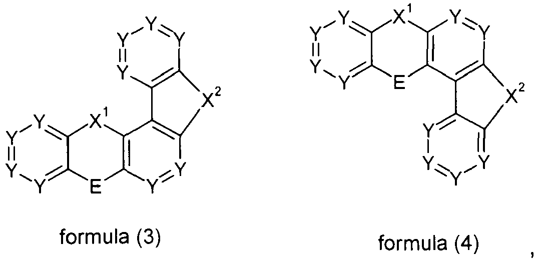 where the following applies to the symbols used:
Y is C if a group X² is bonded to this group Y, or is on each occurrence, identically or differently, CR or N if no group X² is bonded to this group Y;
E is a single bond or a divalent bridge selected from N(R¹), C(R¹)₂, O or S;
X¹, X² are on each occurrence, identically or differently, a divalent bridge selected from N(R¹), O, C(R¹)₂ or S; with the proviso that X¹ and X² do not simultaneously stand for O;
furthermore the following applies to the symbols and indices used in formulae (1), (2), (3) and (4):
R is on each occurrence, identically or differently, H or D or the radicals R that are not equal to H or D are selected from the group consisting of N(Ar)₂, a straight-chain alkyl group having 1 to 10 C atoms, a branched alkyl group having up to 10 C atoms, or an aromatic or heteroaromatic ring system having 5 to 40 ring atoms, which may be substituted by one or more radicals R²;
R¹, if E, X¹ and/or X² stand for NR¹, stands for an aromatic or heteroaromatic ring system having 6 to 24 aromatic ring atoms, which may be substituted by one or more radicals R²; and, if E, X¹ and/or X² stand for C(R¹)₂, stands for a straight-chain alkyl group having 1 to 20 C atoms or a branched or cyclic alkyl group having 3 to 20 C atoms, which may in each case be substituted by one or more radicals R², where one or more H atoms may be replaced by D, F or CN, or for an aromatic or heteroaromatic ring system having 5 to 60 aromatic ring atoms, which may be substituted by one or more radicals R²;
Ar is on each occurrence, identically or differently, an aromatic or heteroaromatic ring system, preferably an aryl or heteroaryl group having 5 to 40 ring atoms, which may be substituted by one or more radicals R³;
R² is on each occurrence, identically or differently, H, D, F, N(Ar)₂, CN, a straight-chain alkyl group having 1 to 40 C atoms or a branched or cyclic alkyl group having 3 to 40 C atoms, where one or more H atoms may be replaced by D, F or CN, or an aromatic or heteroaromatic ring system having 5 to 60 aromatic ring atoms, which may in each case be substituted by one or more radicals R³;
R³ is on each occurrence, identically or differently, H, D or an aliphatic hydrocarbon radical having 1 to 20 C atoms or an aryl or heteroaryl group having 5 to 40 ring atoms or a combination of these groups;
n, m are, identically or differently on each occurrence, 0 or 1, with the proviso that n + m = 1 or 2;
**characterised in that** the compound of the formula (1), (2), (3) or (4) contains at least one group R and/or R¹ which stands for a group of the following formula (5), 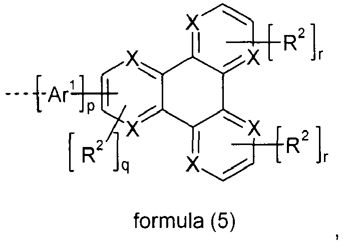 where the dashed bond indicates the linking of the group of the formula (5), R² has the meanings given above and furthermore:
X is C if the group Ar¹ or the remainder of the molecule is bonded to this X, and is on each occurrence, identically or differently, CR² or N in the other cases;
Ar¹ is a divalent aromatic or heteroaromatic ring system having 5 to 40 aromatic ring atoms, which may be substituted by one or more radicals R²;
p is on each occurrence, identically or differently, 0 or 1;
q, r is on each occurrence, identically or differently, 0, 1 or 2.

2. Compound of the formula (2), (3) or (4) according to Claim 1, **characterised in that** X¹ stands for N(R¹) and X² stands for C(R¹)₂ and **in that** E simultaneously stands for a single bond.

3. Compound according to Claim 1 or 2, **characterised in that** Y stands for C if a group X² is bonded to this group Y, or stands, identically or differently on each occurrence, for CR if no group X² is bonded to this group Y.

4. Compound according to one or more of Claims 1 to 3, **characterised in that** X stands for CR², or **in that** X stands for C if the group Ar¹ or the remainder of the molecule is bonded to this X.

5. Compound according to one or more of Claims 1 to 4, **characterised in that** the compound is selected from compounds of the formula (1a), (2a), (3a) or (4a), 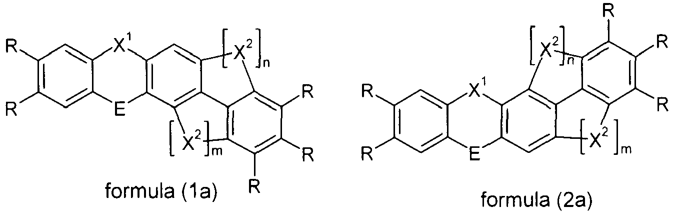 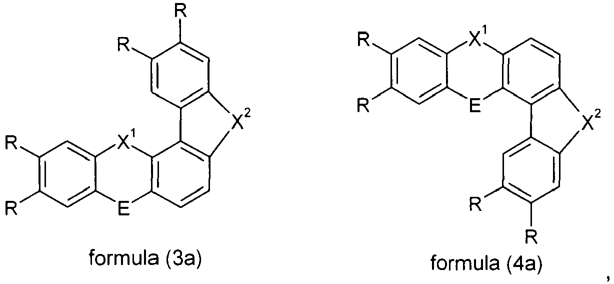 where the symbols and indices used have the meanings given in Claim 1 and X¹ preferably stands for NR¹.

6. Compound according to one or more of Claims 1 to 5, **characterised in that** the compound is selected from compounds of the formula (1e): 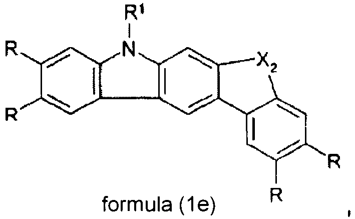 where the symbols and indices have the meanings indicated in Claim 1.

7. Compound according to one or more of Claims 1 to 6, **characterised in that** the group of the formula (5) represents a group of the formula (5a), 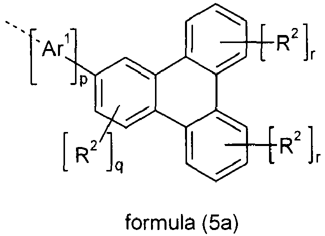 where the symbols and indices used have the meanings given in Claim 1.

8. Compound according to one or more of Claims 1 to 7, **characterised in that** the group of the formula (5) represents a group of the formula (5b) or (5c), 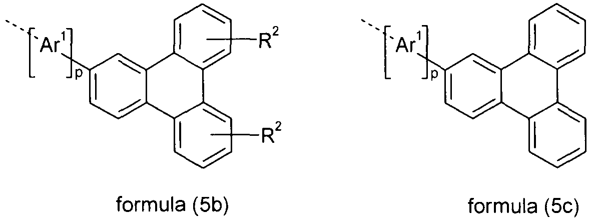 where the symbols and indices used have the meanings given in Claim 1.

9. Process for the preparation of a compound according to one or more of Claims 1 to 8, **characterised by** the steps:
a) synthesis of the skeleton of the formula (1), (2), (3) or (4) which does not yet contain a group of the formula (5); and
b) introduction of the group of the formula (5) by a metal-catalysed coupling reaction.

10. Mixture comprising at least one compound according to one or more of Claims 1 to 8 and at least one further compound, in particular a phosphorescent dopant.

11. Formulation, in particular a solution, a suspension or a mini-emulsion, comprising at least one compound according to one or more of Claims 1 to 8 or a mixture according to Claim 10 and one or more solvents.

12. Use of a compound according to one or more of Claims 1 to 8 or a mixture according to Claim 10 in an electronic device.

13. Electronic device comprising at least one compound according to one or more of Claims 1 to 8 or a mixture according to Claim 10, in particular selected from the group consisting of organic electroluminescent devices, organic integrated circuits, organic field-effect transistors, organic thin-film transistors, organic light-emitting transistors, organic solar cells, organic dye-sensitised solar cells, organic optical detectors, organic photoreceptors, organic field-quench devices, light-emitting electrochemical cells, organic laser diodes and organic plasmon emitting devices.

14. Electronic device according to Claim 13, **characterised in that** it is an organic electroluminescent device and the compound according to one or more of Claims 1 to 8 is employed as matrix material for fluorescent or phosphorescent emitters and/or in a hole-blocking layer and/or in an electron-transport layer and/or in an electron-blocking or exciton-blocking layer and/or in a hole-transport layer.

## Revendications

1. Composé de la formule (1) : 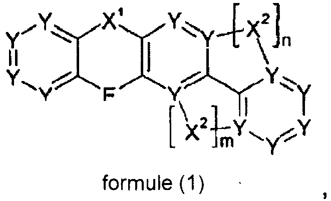 dans laquelle ce qui suit s'applique aux symboles utilisés :
Y est C si un groupe X² est lié à ce groupe Y, ou est, pour chaque occurrence, de manière identique ou différente, CR si aucun groupe X² n'est lié à ce groupe Y ;
E est une liaison simple ;
X¹ est un pont divalent et représente N(R¹) ;
X² est un pont divalent et représente C(R¹)₂ ;
ou composé de la formule (2), de la formule (3) ou de la formule (4) : 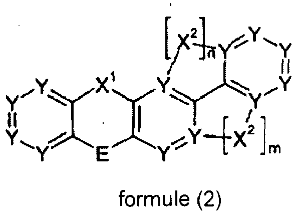 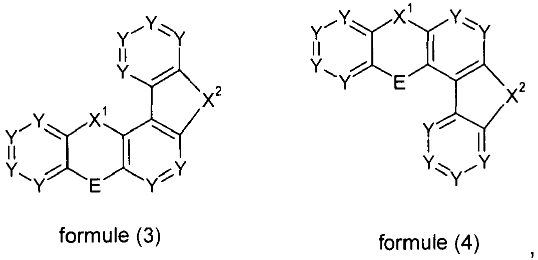 dans lesquelles ce qui suit s'applique aux symboles utilisés :
Y est C si un groupe X² est lié à ce groupe Y, ou est, pour chaque occurrence, de manière identique ou différente, CR ou N si aucun groupe X² n'est lié à ce groupe Y ;
E est une liaison simple ou un pont divalent qui est sélectionné parmi N(R¹), C(R¹)₂, O ou S ;
X¹, X² sont, pour chaque occurrence, de manière identique ou différente, un pont divalent qui est sélectionné parmi N(R¹), O, C(R¹)₂ ou S ; étant entendu que X¹ et X² ne représentent pas simultanément O ;
en outre, ce qui suit s'applique aux symboles et indices utilisés dans les formules (1), (2), (3) et (4) :
R est, pour chaque occurrence, de manière identique ou différente, H ou D ou les radicaux R qui ne sont pas égaux à H ou D sont sélectionnés parmi le groupe qui est constitué par N(Ar)₂, un groupe alkyle en chaîne droite qui comporte 1 à 10 atome(s) de C, un groupe alkyle ramifié qui comporte jusqu'à 10 atomes de C, ou un système de cycle aromatique ou hétéroaromatique qui comporte 5 à 40 atomes de cycle, lequel peut être substitué par un radical ou plusieurs radicaux R² ;
R¹, si E, X¹ et/ou X² représentent NR¹, représente un système de cycle aromatique ou hétéroaromatique qui comporte 6 à 24 atomes de cycle aromatique, lequel peut être substitué par un radical ou plusieurs radicaux R² ;
et, si E, X¹ et/ou X² représentent C(R¹)₂, représente un groupe alkyle en chaîne droite qui comporte 1 à 20 atome(s) de C ou un groupe alkyle ramifié ou cyclique qui comporte 3 à 20 atomes de C, lequel peut dans chaque cas être substitué par un radical ou plusieurs radicaux R², où un ou plusieurs atome(s) de H peut/peuvent être remplacé(s) par D, F ou CN, ou un système de cycle aromatique ou hétéroaromatique qui comporte 5 à 60 atomes de cycle aromatique, lequel peut être substitué par un radical ou plusieurs radicaux R² ;
Ar est, pour chaque occurrence, de manière identique ou différente, un système de cycle aromatique ou hétéroaromatique, de préférence un groupe aryle ou hétéroaryle qui comporte 5 à 40 atomes de cycle, lequel peut être substitué par un radical ou plusieurs radicaux R³;
R² est, pour chaque occurrence, de manière identique ou différente, H, D, F, N(Ar)₂, CN, un groupe alkyle en chaîne droite qui comporte 1 à 40 atome(s) de C ou un groupe alkyle ramifié ou cyclique qui comporte 3 à 40 atomes de C, où un ou plusieurs atome(s) de H peut/peuvent être remplacé(s) par D, F ou CN, ou un système de cycle aromatique ou hétéroaromatique qui comporte 5 à 60 atomes de cycle aromatique, lequel peut, dans chaque cas, être substitué par un radical ou plusieurs radicaux R³;
R³ est, pour chaque occurrence, de manière identique ou différente, H, D ou un radical hydrocarbone aliphatique qui comporte 1 à 20 atome(s) de C ou un groupe aryle ou hétéroaryle qui comporte 5 à 40 atomes de cycle ou une combinaison de ces groupes ;
n, m sont, de manière identique ou différente pour chaque occurrence, 0 ou 1, étant entendu que n + m = 1 ou 2 ;
**caractérisé en ce que** le composé de la formule (1), (2), (3) ou (4) contient au moins un groupe R et/ou R¹ qui représente un groupe de la formule (5) qui suit : 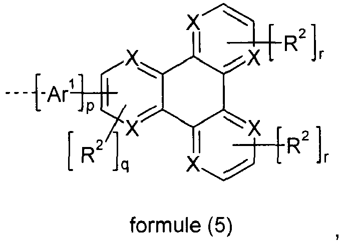 dans laquelle le lien en pointillés indique la liaison du groupe de la formule (5), R² présente les significations qui ont été données ci-avant et en outre :
X est C si le groupe Ar¹ ou le reste de la molécule est lié à ce X, et est, pour chaque occurrence, de manière identique ou différente, CR² ou N dans les autres cas ;
Ar¹ est un système de cycle divalent aromatique ou hétéroaromatique qui comporte 5 à 40 atomes de cycle aromatique, lequel peut être substitué par un radical ou plusieurs radicaux R² ;
p est, pour chaque occurrence, de manière identique ou différente, 0 ou 1 ;
q, r sont, pour chaque occurrence, de manière identique ou différente, 0, 1 ou 2.

2. Composé de la formule (2), (3) ou (4) selon la revendication 1, **caractérisé en ce que** X¹ représente N(R¹) et X² représente C(R¹)₂ et **en ce que** E représente simultanément une liaison simple.

3. Composé selon la revendication 1 ou 2, **caractérisé en ce que** Y représente C si un groupe X² est lié à ce groupe Y, ou représente, de manière identique ou différente pour chaque occurrence, CR si aucun groupe X² n'est lié à ce groupe Y.

4. Composé selon une ou plusieurs des revendications 1 à 3, **caractérisé en ce que** X représente CR², ou **en ce que** X représente C si le groupe Ar¹ ou le reste de la molécule est lié à ce X.

5. Composé selon une ou plusieurs des revendications 1 à 4, **caractérisé en ce que** le composé est sélectionné parmi les composés de la formule (1a), (2a), (3a) ou (4a) : 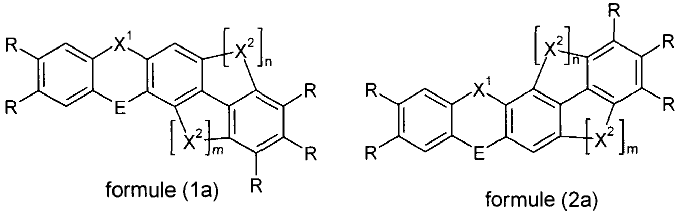 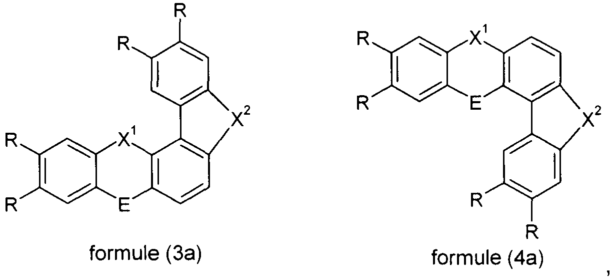 dans lesquelles les symboles et indices utilisés présentent les significations qui ont été données selon la revendication 1 et X¹ représente de préférence NR¹.

6. Composé selon une ou plusieurs des revendications 1 à 5, **caractérisé en ce que** le composé est sélectionné parmi les composés de la formule (1e) : 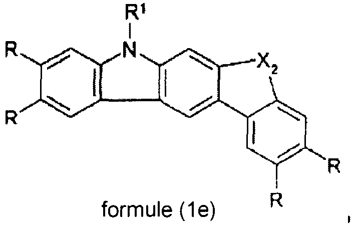 dans laquelle les symboles et indices utilisés présentent les significations qui ont été données selon la revendication 1.

7. Composé selon une ou plusieurs des revendications 1 à 6, **caractérisé en ce que** le groupe de la formule (5) représente un groupe de la formule (5a) : 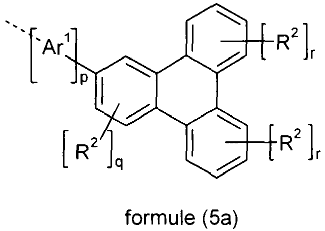 dans laquelle les symboles et indices utilisés présentent les significations qui ont été données selon la revendication 1.

8. Composé selon une ou plusieurs des revendications 1 à 7, **caractérisé en ce que** le groupe de la formule (5) représente un groupe de la formule (5b) ou (5c) : 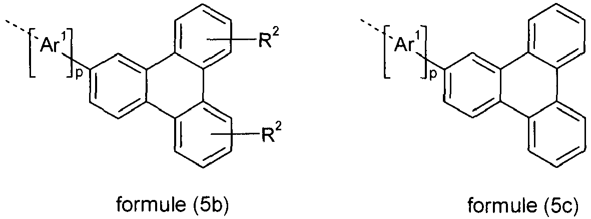 dans lesquelles les symboles et indices utilisés présentent les significations qui ont été données selon la revendication 1.

9. Procédé pour la préparation d'un composé selon une ou plusieurs des revendications 1 à 8, **caractérisé par** les étapes de :
a) synthèse du squelette de la formule (1), (2), (3) ou (4), lequel ne contient pas encore un groupe de la formule (5) ; et
b) introduction du groupe de la formule (5) au moyen d'une réaction de couplage catalysée par métal.

10. Mélange comprenant au moins un composé selon une ou plusieurs des revendications 1 à 8 et au moins un autre composé, en particulier un dopant phosphorescent.

11. Formulation, en particulier une solution, une suspension ou une mini-émulsion, comprenant au moins un composé selon une ou plusieurs des revendications 1 à 8 ou un mélange selon la revendication 10 et un ou plusieurs solvant(s).

12. Utilisation d'un composé selon une ou plusieurs des revendications 1 à 8 ou d'un mélange selon la revendication 10 dans un dispositif électronique.

13. Dispositif électronique comprenant au moins un composé selon une ou plusieurs des revendications 1 à 8 ou un mélange selon la revendication 10, en particulier sélectionné parmi le groupe qui est constitué par les dispositifs électroluminescents organiques, les circuits intégrés organiques, les transistors à effet de champ organiques, les transistors à film mince organiques, les transistors à émission de lumière organiques, les cellules solaires organiques, les cellules solaires sensibilisées par colorant organiques, les détecteurs optiques organiques, les photorécepteurs organiques, les dispositifs à extinction de champ organiques, les cellules électrochimiques à émission de lumière, les diodes laser organiques et les dispositifs à émission de plasmons organiques.

14. Dispositif électronique selon la revendication 13, **caractérisé en ce qu'**il s'agit d'un dispositif électroluminescent organique et le composé selon une ou plusieurs des revendications 1 à 8 est utilisé en tant que matériau de matrice pour des émetteurs fluorescents ou phosphorescents et/ou dans une couche de blocage de trous et/ou dans une couche de transport d'électrons et/ou dans une couche de blocage d'électrons ou une couche de blocage d'excitons et/ou dans une couche de transport de trous.
